# EUROPÄISCHE PATENTSCHRIFT

(11) **EP 0 582 908 B1**
(45) Veröffentlichungstag und Bekanntmachung des Hinweises auf die Patenterteilung: **27.05.1998**
(21) Anmeldenummer: 93112154.5
(22) Anmeldetag: 29.07.1993
(51) Int. Cl.: C07D 215/14, A61K 31/47

(54) **2-Substituierte Chinolylmethoxy-phenylessigsäure- und -benzylacyl Derivate, Verfahren zu ihrer Herstellung und ihre Verwendung als Leukotriensynthese Hemmer**
2-Substituted quinolylmethoxy-phenylacetic acid- and -benzylacyl derivatives, process for their preparation and their use as inhibitors of leukotriene synthesis
Dérivés d'acide quinoléinylméthoxy-phénylacétique et - benzylacyle 2-substitués, procédé pour leur préparation et leur utilisation comme inhibiteurs de la synthèse de leukotriène

(30) Priorität: 11.08.1992 DE 4226519
(43) Veröffentlichungstag der Anmeldung: 16.02.1994
(73) Patentinhaber: BAYER AG, 51368 Leverkusen (DE)
(72) Erfinder: Matzke, Michael, Dr., D-42113 Wuppertal (DE); Mohrs, Klaus-Helmut, Dr., D-42113 Wuppertal (DE); Raddatz, Siegfried, Dr., D-51065 Köln (DE); Fruchmann, Romanis, Dr., D-50735 Köln (DE); Müller-Peddinghaus, Rainer, Prof. Dr., D-51467 Bergisch Gladbach (DE); Hatzelmann, Armin, Dr., D-78467 Konstanz (DE)

(56) Entgegenhaltungen:
- EP-A- 0 339 416
- EP-A- 0 344 519
- EP-A- 0 399 291
- EP-A- 0 414 076
- EP-A- 0 414 078
- EP-A- 0 545 170

## Beschreibung

Die vorliegende Erfindung betrifft 2-substituierte Chinolyl-methoxy-phenylessigsäurederivate, Verfahren zu ihrer Herstellung sowie ihre Verwendung in Arzneimitteln.

Substituierte 4-(Chinolin-2-yl-methoxy)phenylessigsäurederivate und α-substituierte 4-(Chinolin-2-yl-methoxy)phenylessigsäurederivate sind, wie beispielsweise Phenylessigsäuren, Phenylessigsäureester, Phenylessigsäureamide, sowie Sulfonamide sind aus EP 545 170, EP 414078, EP 414 076, EP 399 291, EP 344 519 und EP 339 416 bekannt. Die Verbindungen, die in diesen Dokumenten offenbart sind, sind in der zentralen Phenyleinheit unsubstituiert.

Die vorliegende Erfindung betrifft jetzt 2-substituierte Chinolylmethoxy-phenylessigsäure- und -benzylacyl-derivate der allgemeinen Formel (I) 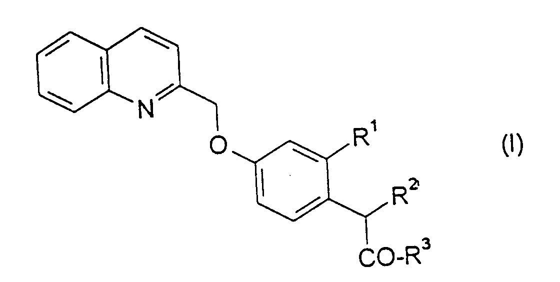 in welcher
- R¹: für Halogen, Cyano, Nitro, Azido, Hydroxy, Carboxy, Trifluoromethyl, Trifluoromethoxy oder Trifluoromethylthio steht, oder für geradkettiges oder verzweigtes Alkyl, Alkenyl oder Alkinyl mit jeweils bis zu 8 Kohlenstoffatomen steht, die gegebenenfalls durch Phenyl oder Cycloalkyl mit 3 bis 8 Kohlenstoffatomen substituiert sind, oder für Cycloalkyl mit 3 bis 8 Kohlenstoffatomen oder Phenyl steht, oder für geradkettiges oder verzweigtes Alkoxy oder Alkoxycarbonyl mit jeweils bis zu 6 Kohlenstoffatomen steht,
- R²: für Wasserstoff oder für geradkettiges oder verzweigtes Alkyl mit bis zu 6 Kohlenstoffatomen steht, oder für Cycloalkyl mit 3 bis 12 Kohlenstoffatomen steht,
- R³: für Hydroxy, für geradkettiges oder verzweigtes Alkoxy mit bis zu 8 Kohlenstoffatomen oder Phenyl steht, oder
für eine Gruppe der Formel -NR⁴SO₂R⁵ oder -NR⁶R⁷ steht,
worin
- R⁴, R⁶ und R⁷: gleich oder verschieden sind und Wasserstoff, geradkettiges oder verzweigtes Alkyl mit bis zu 6 Kohlenstoffatomen, Phenyl oder Benzyl bedeuten,
- R⁵: Trifluormethyl oder Phenyl bedeutet, das gegebenenfalls durch Halogen, Cyano, Hydroxy, Nitro Trifluormethyl, Trifluormethoxy, Trifluormethylthio oder durch geradkettiges oder verzweigtes Alkyl oder Alkoxy mit jeweils bis zu 6 Kohlenstoffatomen substituiert ist, oder
geradkettiges oder verzweigtes Alkyl mit bis zu 8 Kohlenstoffatomen bedeutet, das gegebenenfalls durch Phenyl substituiert ist, das seinerseits durch Halogen, Cyano, Nitro, Trifluormethyl, Trifluormethoxy, Trifluoromethylthio, Hydroxy oder durch geradkettiges oder verzweigtes Alkyl oder Alkoxy mit jeweils bis zu 6 Kohlenstoffatomen substituiert sein kann
und deren Salze.

Im Rahmen der vorliegenden Erfindung werden physiologisch unbedenkliche Salze bevorzugt. Physiologisch unbedenkliche Salze der (Chinolin-2-ylmethoxy)-phenyl-essigsäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Die erfindungsgemäßen Verbindungen existieren in stereoisomeren Formen (*), die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher
- R¹: für Fluor, Chlor, Brom, Iod, Cyano, Nitro, Azido, Hydroxy, Carboxy, Trifluoromethyl oder Trifluormethoxy steht, oder für geradkettiges oder verzweigtes Alkyl, Alkenyl oder Alkinyl mit jeweils bis zu 6 Kohlenstoffatomen steht, die gegebenenfalls durch Phenyl, Cyclopropyl, Cyclopentyl oder Cyclohexyl substituiert sind, oder für Cyclopropyl, Cyclobutyl, Cyclopentyl, Cyclohexyl oder Cycloheptyl oder Phenyl steht, oder für geradkettiges oder verzweigtes Alkoxy oder Alkoxycarbonyl mit jeweils bis zu 4 Kohlenstoffatomen steht,
- R²: für Wasserstoff oder geradkettiges oder verzweigtes Alkyl mit bis zu 4 Kohlenstoffatomen steht, oder für Cyclopropyl, Cyclobutyl, Cyclopentyl, Cyclohexyl, Cycloheptyl oder Cyclooctyl steht,
- R³: für Hydroxy oder für geradkettiges oder verzweigtes Alkoxy mit bis zu 6 Kohlenstoffatomen oder Phenyl steht, oder
für eine Gruppe der Formel -NR⁴SO₂R⁵ oder -NR⁶R⁷ steht,
worin
- R⁴, R⁶ und R⁷: gleich oder verschieden sind und Wasserstoff oder geradkettiges oder verzweigtes Alkyl mit bis zu 4 Kohlenstoffatomen bedeuten,
- R⁵: Trifluormethyl oder Phenyl bedeutet, das gegebenenfalls durch Fluor, Chlor, Brom, Iod, Cyano oder durch geradkettiges oder verzweigtes Alkyl oder Alkoxy mit jeweils bis zu 4 Kohlenstoffatomen substituiert ist, oder
geradkettiges oder verzweigtes Alkyl mit bis zu 6 Kohlenstoffatomen bedeutet, das gegebenenfalls durch Phenyl substituiert ist, das seinerseits durch Fluor, Chlor, Brom, Trifluormethyl oder durch geradkettiges oder verzweigtes Alkyl oder Alkoxy mit jeweils bis zu 4 Kohlenstoffatomen substituiert sein kann
und deren Salze.

Besonders bevorzugt sind Verbindungen der allgemeinen Formel (I),
in welcher
- R¹: für Fluor, Chlor, Brom, Nitro, Azido oder Trifluormethoxy steht, oder für geradkettiges oder verzweigtes Alkyl, Alkenyl oder Alkinyl mit jeweils bis zu 4 Kohlenstoffatomen steht, die gegebenenfalls durch Phenyl oder Cyclopropyl substituiert sind, oder
für Cyclopropyl, Cyclopentyl oder Cyclohexyl steht,
- R²: für Wasserstoff oder für geradkettiges oder verzweigtes Alkyl mit bis zu 3 Kohlenstoffatomen steht, oder
für Cyclopentyl, Cyclohexyl oder Cycloheptyl steht,
- R³: für Hydroxy oder für geradkettiges oder verzweigtes Alkoxy mit bis zu 4 Kohlenstoffatomen steht, oder
für eine Gruppe der Formel -NR⁴SO₂R⁵ oder -NR⁶R⁷ steht,
worin
- R⁴, R⁶ und R⁷: gleich oder verschieden sind und Wasserstoff oder Methyl bedeuten,
- R⁵: Trifluormethyl oder Phenyl bedeutet, das gegebenenfalls durch Fluor, Chlor, Brom, Jod, Methoxy, Methyl oder Trifluormethyl substituiert ist, oder
geradkettiges oder verzweigtes Alkyl mit bis zu 4 Kohlenstoffatomen bedeutet, das gegebenenfalls durch Phenyl substituiert ist, das seinerseits durch Fluor, Chlor, Brom, Methyl oder Methoxy substituiert sein kann
und deren Salze.

Außerdem wurden Verfahren zur Herstellung der erfindungsgemäßen Verbindungen der allgemeinen Formel (I) gefunden, dadurch gekennzeichnet, daß man
[A] Phenole der allgemeinen Formel (II) 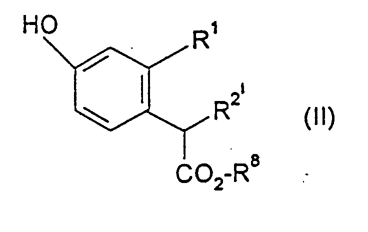 in welcher
   - R¹: die oben angegebene Bedeutung hat,
   - R^{2'}: die oben angegebene Bedeutung von R² hat aber nicht für Wasserstoff steht,
   - R⁸: für C₁-C₄-Alkyl steht,
   mit 2-Halogenmethylchinolinen der allgemeinen Formel (III) 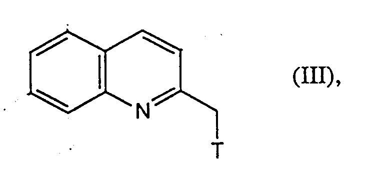 in welcher
   - T: für Halogen, vorzugsweise für Chlor oder Brom steht,
   in inerten Lösemitteln verethert, oder
[B] Phenole der allgemeinen Formel (IIa) 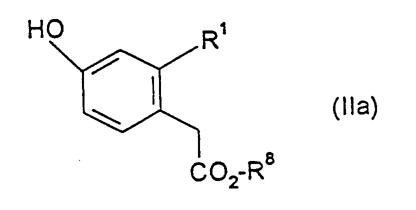 in welcher
   - R¹ und R⁸: die oben angegebene Bedeutung haben,
   zunächst durch Umsetzung mit den Verbindungen der allgemeinen Formel (III) in inerten Lösemitteln in die Verbindungen der allgemeinen Formel (Ia) 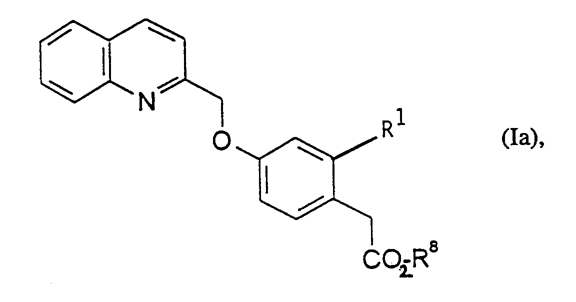 in welcher
   - R¹ und R⁸: die oben angegebene Bedeutung haben, überführt,
   und diese anschließend mit Verbindungen der allgemeinen Formel (IV)

   R^{2'}-W (IV),

   in welcher
   - R^{2'}: die oben angegebene Bedeutung hat
   und
   - W: für Chlor, Brom oder Jod steht,
   in inerten Lösemitteln alkyliert,
   und im Fall der Säuren (R³ = OH) die Ester verseift,
   im Fall, daß R³ für die Gruppe der Formel -NR⁴SO₂R⁵ oder -NR⁶R⁷ steht, die Säuren (R³ = OH), gegebenenfalls unter vorgeschalteter Aktivierung, diese entweder mit den entsprechenden Sulfonamiden der Formel (V), den Aminen oder Ammoniak der Formel (VI)

   NHR⁴SO₂-R⁵ (V)

   oder

   HNR⁶R⁷ (VI),

   in welcher
   - R⁴, R⁵, R⁶ und R⁷: die oben angegebene Bedeutung haben,
   sulfoamidiert bzw. amidiert.

im Fall, daß R¹ für Alkenyl oder Alkinyl steht, ausgehend von den entsprechenden Halogenverbindungen der allgemeinen Formel (Ia / R¹ = Halogen, vorzugsweise Brom) mit Verbindungen der allgemeinen Formel (VII) (

C₄H₉)₃Sn-R^{1'} (VII),

in welcher
- R^{1'}: für Alkenyl oder Alkinyl mit bis zu 8 C-Atomen steht,
in Anwesenheit von Palladium(0)-Katalysatoren, vorzugsweise Tetrakis(triphenylphosphin)palladium(0), umsetzt,
und im Fall, daß R¹ = (C₁-C₈)-Alkyl, gegebenenfalls anschließend nach üblichen Methoden hydriert,
und im Fall der Enantiomere die entsprechenden enantiomerenreinen Säuren (I / R³ = OH) nach üblicher Methode trennt,
und den Substituenten R¹ gegebenenfalls auf jeder der oben aufgeführten Stufen durch weitere übliche Methoden einführt oder variiert.

Die erfindungsgemäßen Verfahren können durch folgendes Formelschema beispielhaft erläutert werden:

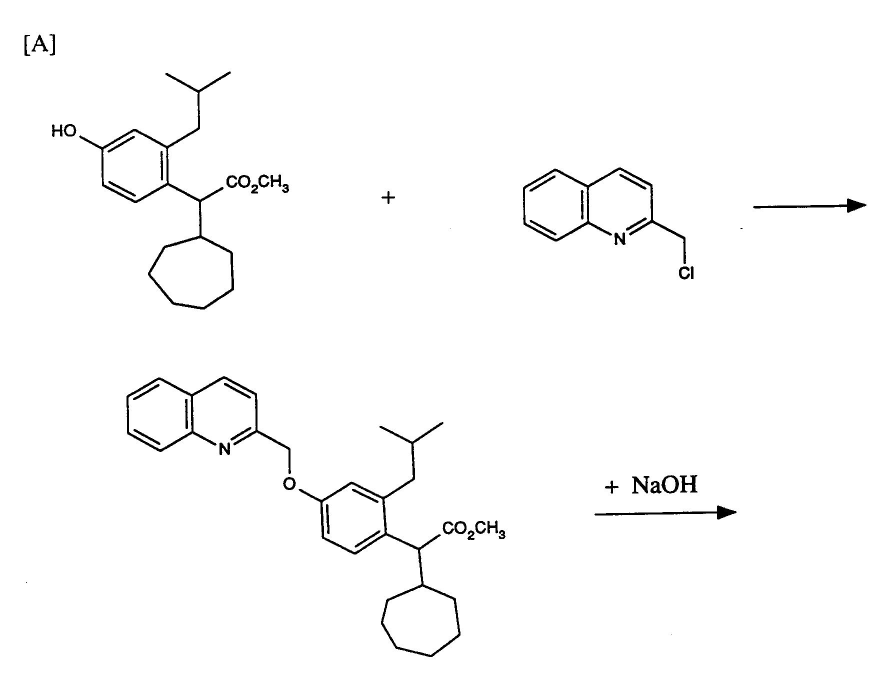

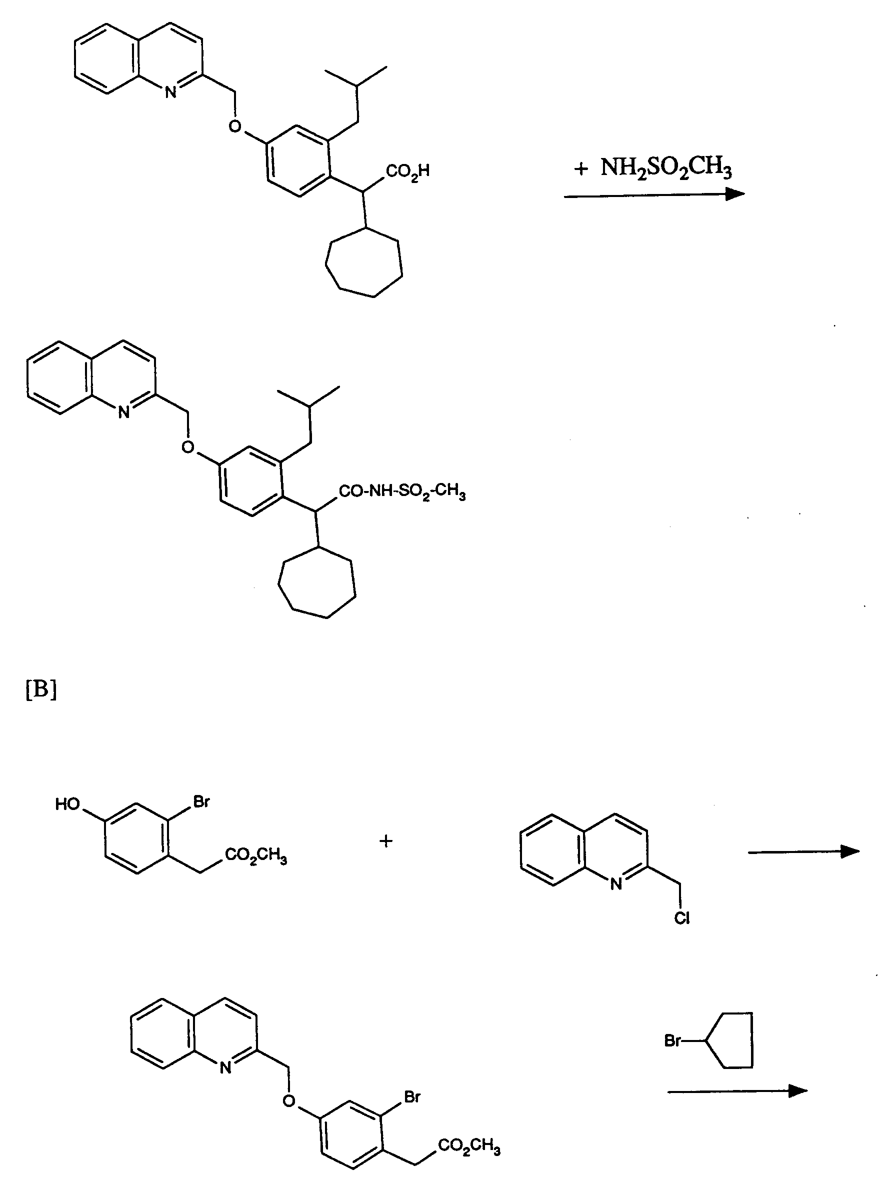

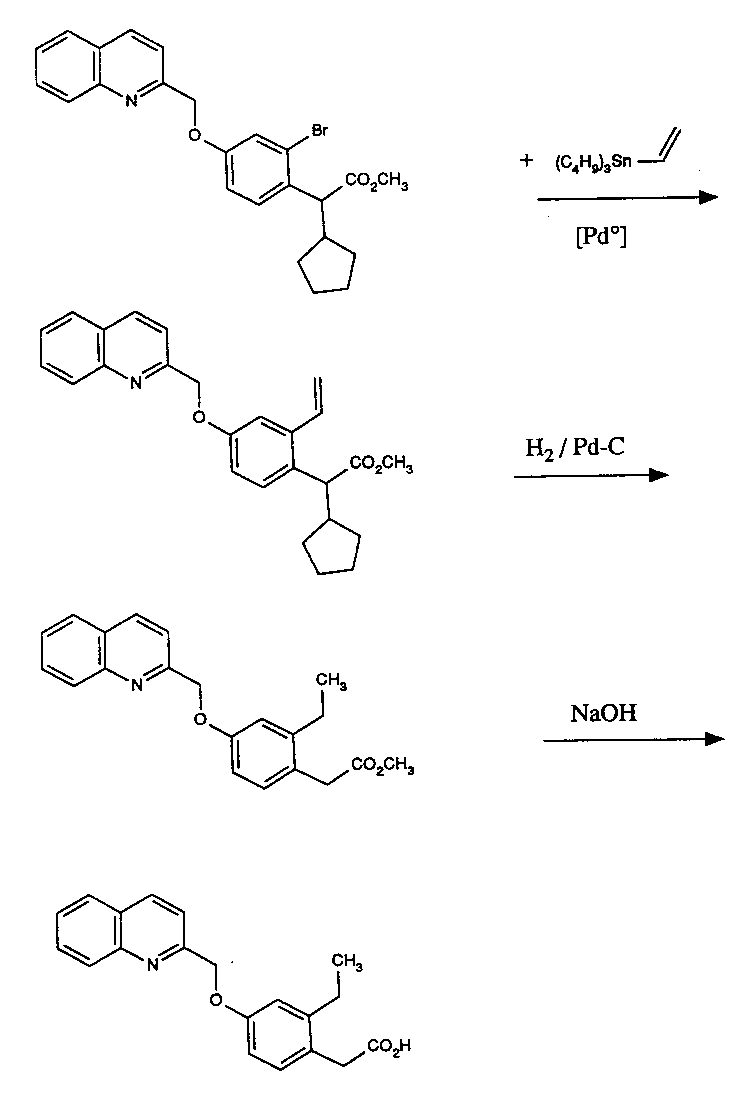

Die Veretherung kann in inerten organischen Lösemitteln gegebenenfalls in Anwesenheit einer Base durchgeführt werden. Lösemittel für die Veretherung können inerte organische Lösemittel sein, die sich unter den Reäktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie z.B. Natriumhydroxid oder Kaliumhydroxid, Erdalkalihydroxide wie z.B. Bariumhydroxid, Alkalicarbonate wie Natriumcarbonat oder Kaliumcarbonat, Erdalkalicarbonate wie Calciumcarbonat, oder organische Amine (Trialkyl(C₁-C₆)amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhydrid, einzusetzen.

Die Veretherung erfolgt im allgemeinen in einem Temperaturbereich von 0°C bis +150°C, bevorzugt von +10°C bis +100°C.

Die Veretherung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5 Mol, bevorzugt 1 bis 2 Mol Halogenid (III), bezogen auf 1 Mol des Reaktionspartners, ein. Die Base wird im allgemeinen in einer Menge von 0,5 bis 5 Mol, bevorzugt von 1 bis 3 Mol, bezogen auf das Halogenid, eingesetzt.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Amidierung und die Sulfoamidierung erfolgten im allgemeinen in inerten Lösemitteln in Anwesenheit einer Base und eines Dehydratisierungsmittels.

Als Lösemittel eignen sich hierbei inerte organische Lösemittel, die sich unter den Reäktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Amidierung und die Sulfoamidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.butylat, oder organische Amine wie Benzyltrimethylammoniumhydroxid, Tetrabutylammoniumhydroxid, Pyridin, Triethylamin oder N-Methylpiperidin.

Die Amidierung und die Sulfoamidierung werden im allgemeinen in einem Temperaturbereich von 0°C bis 150°C, bevorzugt bei 25°C bis 40°C, durchgeführt.

Die Amidierung und die Sulfoamidierung werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Amidierung und der Sulfoamidierung wird die Base im allgemeinen in einer Menge von 1 bis 3 Mol, bevorzugt von 1 bis 1,5 Mol, bezogen auf 1 Mol der jeweiligen Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochiorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroforrnat oder Benzotriazolyloxy-tris-(dimethylamino)phosphonium-hexafluorophosphat oder Phosphorsäurediphenylesterazid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Insbesondere hat es sich als zweckmäßig erwiesen, die Umsetzung im Ammoniakstrom (R⁶R⁷ = H), mit leichtem Überdruck, durchzuführen.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Teträhydrofüran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reäktanden.

Die Hydrierung erfolgt im allgemeinen in inerten Lösemitteln wie Alkoholen, wie beispielsweise Methanol, Ethanol, Propanol oder Isopropanol, vorzugsweise in Methanol, in Gegenwart eines Edelmetallkatalysators wie Platin, Palladium, Palladium auf Tierkohle oder Raney-Nickel, in einem Temperaturbereich von 0°C bis +150°C, bevorzugt von Raumtemperatur bis +100°C, bei Normaldruck oder bei Überdruck möglich.

Die reinen Enantiomeren der erfindungsgemäßen Verbindungen der allgemeinen Formel (I) können beispielsweise hergestellt werden, indem man die entsprechenden Säurenracemate nach üblicher Methode in die Enantiomeren trennt und diese anschließend wie oben aufgeführt weiter umsetzt.

Die Phenole der allgemeinen Formel (II) und (IIa) sind bekannt oder, insbesondere im Fall, daß R¹ für Alkyl steht, als konkrete Stoffvertreter neu und können beispielsweise hergestellt werden, indem man Verbindungen der allgemeinen Formel (VIII) 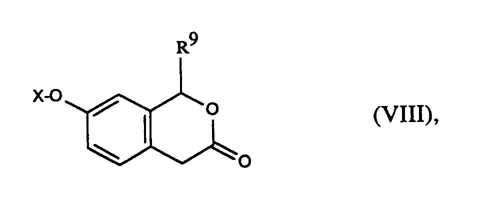 in welcher
- X: für eine Hydroxyschutzgruppe, wie beispielsweise Benzyl, tert Butyl oder auch für Methyl steht
und
- R⁹: für geradkettiges oder verzweigtes Alkyl mit bis zu 7 Kohlenstoffatomen steht,
im Fall, der Verbindungen der allgemeinen Formel (IIa / R² = H), zunächst entweder durch Hydrierung, bevorzugt durch Umsetzung mit Wasserstoff/Pd-C eine Ringöffnung durchführt, anschließend die Hydroxyfunktion nach üblichen Methoden, beispielsweise mit HBr, freisetzt und in einem letzten Schritt mit dem entsprechenden Alkohol, in Anwesenheit einer Säure, vorzugsweise Schwefelsäure, verestert,
und im Fall, der Verbindungen der allgemeinen Formel (II / R^{2'} ≠ H), die Verbindungen der allgemeinen Formel (VIII) zunächst mit Verbindungen der oben aufgeführten allgemeinen Formel (IV) durch Alkylierung in die Verbindungen der allgemeinen Formel (IX) 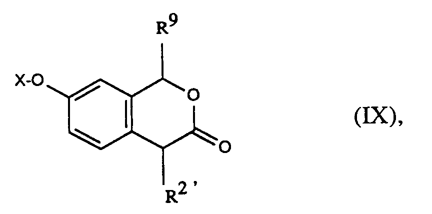 in welcher
- X, R^{2'} und R⁹: die oben angegebene Bedeutung haben,
überführt und anschließend die Ringöffnung, Abspaltung der Schutzgruppe und Veresterung wie oben beschrieben durchführt.

Die Alkylierung und die Hydrierung erfolgen unter den bereits oben aufgeführten Bedingungen.

Die Umsetzung mit Wasserstoff/Pd-C erfolgt im allgemeinen in einem der oben aufgeführten Lösemitteln, vorzugsweise Methanol, in einem Temperaturbereich von 0°C bis 70°C, vorzugsweise von 10°C bis 50°C und einem Druck von 1 bar.

Die Veresterung erfolgt im allgemeinen mit den entsprechenden Alkoholen in Anwesenheit von Säuren, vorzugsweise Schwefelsäure, in einem Temperaturbereich von 0°C bis 150°C, vorzugsweise von 50°C bis 100°C und Normaldruck.

Die Verbindungen der allgemeinen Formel (VIII) sind teilweise bekannt oder können nach bekannten Methoden hergestellt werden, indem man beispielsweise ausgehend von den geschützten 4-Hydroxybenzyldialkylaminen eine Umsetzung mit den entsprechenden Aldehyden in Anwesenheit von Basen, vorzugsweise Butyllithium, in einem der oben aufgeführten Lösemitteln, vorzugsweise Diethylether, zu den entsprechenden substituierten 2-Hydroxymethylbenzyldialkylverbindungen durchführt und anschließend mit Chlorameisensäureester, Kaliumcyanid und Kaliumhydroxid zur entsprechenden 1-substituierten Isochromanonverbindungen cyclisiert.

Die Verbindungen der allgemeinen Formel (IX) sind größtenteils neu und können beispielweise nach dem oben aufgeführten Verfahren hergestellt werden.

Die Abspaltung der Schutzgruppen aus den entsprechenden Ethern (VIII) und (IX) erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas.

Die Verbindungen der allgemeinen Formel (III) sind bekannt oder können nach bekannten Methoden hergestellt werden.

Die Verbindungen der allgemeinen Formel (Ia) sind neu und können wie oben beschrieben hergestellt werden.

Die Verbindungen der allgemeinen Formeln (IV), (V), (VI) und (VII) sind an sich bekannt.

Die erfindungsgemäßen Verbindungen können als Wirkstoffe in Arzneimitteln eingesetzt werden. Die Stoffe können als Hemmer von enzymatischen Reaktionen im Rahmen des Arachidonsäurestoffwechsels, insbesondere der 5-Lipoxygenase, wirken.

Die Verbindungen der allgemeinen Formel (I) zeigen überraschenderweise eine hohe in vitro Aktivität als Leukotriensynthesehemmer und eine starke in vivo Wirkung nach oraler Applikation.

Sie sind somit bevorzugt zur Behandlung und Verhütung von Entzündungen, insbesondere von Erkrankungen der Atemwege wie Allergien/Asthma, Bronchitis, Emphysema, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen Verbindungen können sowohl in der Humanmedizin als auch in der Veterinärmedizin angewendet werden.

Die pharmakologischen Wirkdaten der erfindungsgemäßen Substanzen werden durch folgende Methode bestimmt:

Als Maß für die 5-Lipoxygenase-Hemmung in vitro wurde die Freisetzung von Leukotrien B₄ (LTB₄) an polymorphkernigen Humanleukozyten (PMN) nach Zugabe von Substanzen und Ca-Ionophor mittels reverse phase HPLC nach Borgeat, P. et al., Proc. Nat. Acad. Sci. 76, 2148-2152 (1979), bestimmt.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-%, der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament der Art und Schwere der Erkrankung, der Art der Zubereitung und Applikation, sowie dem Zeitpunkt bzw. Intervall, zu welchem die Verabreichung erfolgt.

### Ausgangsverbindungen

### Beispiel I

### 2-Brom-4-hydroxyphenylessigsäuremethylester

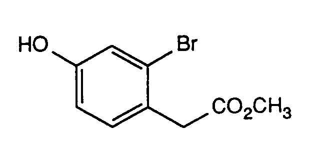

Eine Lösung aus 70 g (0,303 mol) 2-Brom-4-hydroxyphenylessigsäure in 560 ml Methanol und 14 ml Schwefelsäure wird 3 h bei 80°C gerührt. Anschließend wird im Vakuum eingeengt und der Rückstand in Dichlormethan aufgenommen. Die organische Phase wird nacheinander mit Wasser, gesättigter Natriumhydrogencarbonatlösung und Wasser gewaschen, über Natriumsulfat getrocknet und im Vakuum eingeengt. Das Rohprodukt wird säulenchromatographisch gereinigt (Dichlormethan / Methanol, 20:1)
Ausbeute: 54,3 g (73,1% der Theorie)

### Beispiel II

### N-[2-(1-Hydroxyisobutyl)-4-methoxy]benzyldimethylamin

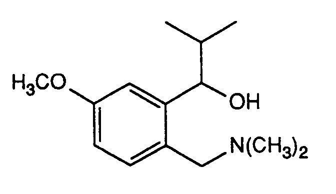

Zu einer Mischung aus 12,5 g (0,075 mol) p-Methoxybenzyldimethylamin mit 60 ml Diethylether p.a. werden unter Argonatmosphäre bei 0°C 140 ml einer 1,6 molaren Lösung von Butyllithium in Hexan (0,224 mol) getropft. Der Ansatz wird 24 h bei Raumtemperatur gerührt. Dann werden 21,5 g (0,298 mol) iso-Butyraldehyd zugetropft, so daß die Reaktionsmischung unter Rückfluß siedet. Anschließend wird 2 h bei Raumtemperatur gerührt, mit 150 ml Wasser versetzt und die organische Phase mit halbkonzentrierter Salzsäure extrahiert. Das Produkt wird aus der wäßrigen Phase nach Versetzen mit 2 N NaOH bis pH 12 mit Ether extrahiert. Die organische Phase wird über Natriumsulfat getrocknet, im Vakuum eingeengt und der Rückstand über Kieselgel 60 (Dichlormethan / Methanol, 9:1) gereinigt.
Ausbeute: 13,7 g (77% der Theorie), Öl.

### Beispiel III

### 1-Isopropyl-7-methoxy-3-isochromanon

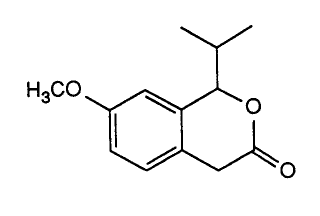

Zu einem Gemisch von 23,7 g (0,1 mol) der Verbindung aus Beispiel II und 60 g Natriumhydrogencarbonat in 375 ml Toluol p.a. wird bei Raumtemperatur eine Lösung aus 148 ml (1,55 mol) Chlorameisensäureethylester in 200 ml Toluol p.a. getropft Es wird 1 h bei Raumtemperatur gerührt. Anschließend wird filtriert und das Filtrat im Vakuum eingeengt. Der Rückstand wird mit 90 ml DMF p.a. und 20 g (0,3 mol) Kaliumcyanid vesetzt und die Reaktionsmischung 6 h bei Raumtemperatur gerührt. Danach wird mit Wasser versetzt, mit Ether extrahiert, die organische Phase über Na₂SO₄ getrocknet und im Vakuum eingeengt. Eine Mischung des Rückstandes in 45 ml Methanol, 15 g Kaliumhydroxid und 100 ml Wasser wird 8 h unter Rückfluß erhitzt. Nach Abdestillation des Lösemittels im Vakuum wird Wasser zugegeben und mit Ether gewaschen. Die wäßrige Phase wird mit halbkonzentrierter Salzsäure angesäuert und das Produkt mit Ether extrahiert. Die organische Phase wird über Natriumsulfat getrocknet und im Vakuum eingeengt. Das Produkt wird säulenchromatographisch über Kieselgel 60 (Petrolether / Ether, 1:1) gereinigt.
Ausbeute: 12,3 g (55,8% der Theorie), Öl.

### Beispiel IV

### 4-Cycloheptyl-1-isopropyl-7-methoxy-3-isochromanon

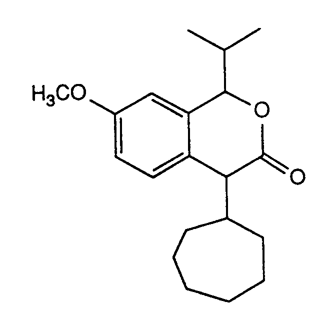

5,3 g (0,024 mol) der Verbindung aus Beispiel III und 8,5 g (0,048 mol) Cycloheptylbromid werden in 25 ml DMF p.a. gelöst. Unter Argonatmosphäre wird bei 0°C eine Lösung aus 5,9 g (0,053 mol) Kalium-tert.burylat zugetropft. Der Reaktionsansatz wird 20 h gerührt, anschließend mit Eiswasser versetzt und mit halbkonzentrierter Salzsäure auf pH 5-6 angesäuert. Es wird mit Ethylacetat extrahiert, die organische Phase über Natriumsulfat getrocknet und im Vakuum eingeengt. Der Rückstand wird an Kieselgel 60 chromatographiert (Petrolether / Ether, 1:1)
Ausbeute: 3,5 g (46% der Theorie), Öl.

### Beispiel V

### 2-Isobutyl-4-methoxyphenylessigsäure

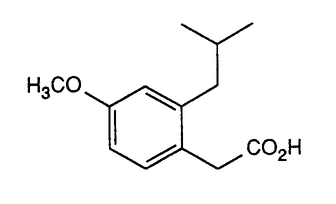

4,6 g (0,02 mol) der Verbindung aus Beispiel III werden in 200 ml Methanol p.a. nach Zugabe von 1 g Pd-Kohle (10%) 5 h bei 1 bar hydriert. Es wird vom Katalysator abfiltriert und das Filtrat im Vakuum eingeengt. Der Rückstand wird in Ether aufgenommen und die organische Phase mit 3%iger Natronlauge extrahiert. Die alkalische wäßrige Phase wird unter Kühlung mit konzentrierter Salzsäure angesäuert und das Produkt mit Ether extrahiert. Nach Trocknen über Natriumsulfat wird die organische Produktphase im Vakuum eingeengt.
Ausbeute: 2,8g (64% der Theorie), Öl.

### Beispiel VI

### 2-Cycloheptyl-2-(2-isobutyl-4-methoxyphenyl)essigsäure

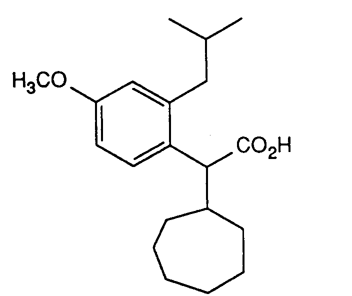

In Analogie zur Vorschrift des Beispiels V wird die Titelverbindung durch Hydrierung von 4,3 g (0,014 mol) der Verbindung aus Beispiel IV in Gegenwart von 1 g Palladium-Kohle (10%) hergestellt.
Ausbeute: 1,8 g (41,6% der Theorie)
Fp.: 119°C

### Beispiel VII

### 4-Hydroxy-2-isobutylphenylessigsäure

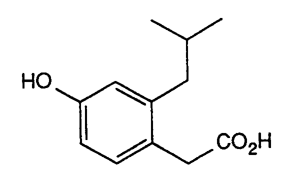

Ein Gemisch aus 2,6 g (0,012 mol) der Verbindung aus Beispiel V und 50 ml 8,8 N (48%iger) Bromwasserstoffsäure wird 1 h unter Rückfluß erhitzt. Nach dem Abkühlen wird mit Wasser verdünnt und das Produkt mit Ethylacetat extrahiert. Die organische Phase wird über Natriumsulfat getrocknet und im Vakuum eingeengt.
Ausbeute: 2,4g Rohprodukt, das direkt zur nächsten Umsetzung eingesetzt wird.

### Beispiel VIII

### 2-Cycloheptyl-2-(4-hydroxy-2-isobutylphenyl)essigsäure

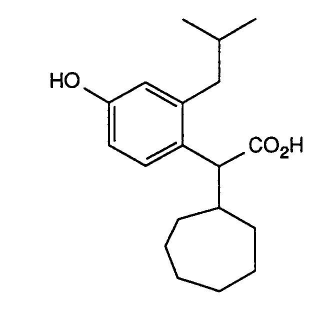

Ein Gemisch aus 3,9 g (0,012 mol) der Verbindung aus Beispiel VI, 25 ml 8,8 N (48%iger) Bromwasserstoffsäure und 25 ml Eisessig wird 3 h unter Rückfluß erhitzt. Anschließend wird mit Wasser verdünnt und mit Ethylacetat extrahiert. Die organische Phase wird mit Wasser gewaschen, über Natriumsulfat getrocknet und im Vakuum eingeengt. Das Produkt wird säulenchromatographisch an Kieselgel 60 gereinigt (Petrolether / Ether 1:1)
Ausbeute: 3,4g (91,2% der Theorie), Öl

### Beispiel IX

### 4-Hydroxy-2-isobutylphenylessigsäuremethylester

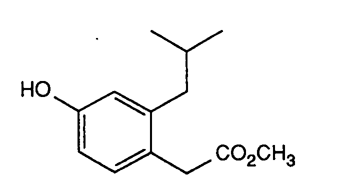

2,4 g des Rohproduktes aus der Verbindung des Beispiels VII werden nach Zugabe von 30 ml Methanol p.a. und 0,7 ml konz. Schwefelsäure 3 h unter Rückfluß erhitzt. Nach Verdünnung mit Wasser wird mit Ethylacetat extrahiert, die organische Phase getrocknet und im Vakuum eingeengt. Der Rückstand wird an Kieselgel 60 chromatographiert (Dichlormethan / Methanol, 50:1).
Ausbeute: 1,7 g (65,4% der Theorie, bezogen auf die Verbindung aus Beispiel V).

### Beispiel X

### 2-Cycloheptyl-2-(4-hydroxy-2-isobutylphenyl)essigsäuremethylester

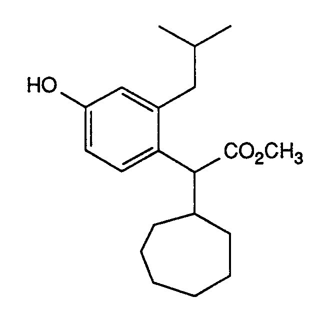

In Analogie zur Vorschrift des Beispiels IX wird die Titelverbindung aus 2 g (7 mmol) der Verbindung aus Beispiel VIII hergestellt.
Ausbeute: 1,8 g (86% der Theorie), Öl

### Herstellungsbeispiele

### Beispiel 1

### 2-Brom-4-(chinolin-2-yl-methoxy)phenylessigsäuremethylester

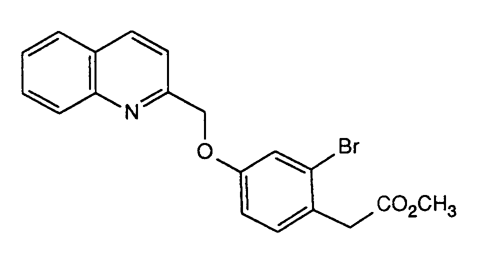

Ein Gemisch aus 14,7 g (0,06 mol) 2-Brom-4-hydroxy-phenylessigsäuremethylester und 20,7 g (0,15 mol) Kaliumcarbonat in 150 ml DMF p.a. wird 1,5 h bei 100°C gerührt. Nach Zugabe von 12,8 g (0,06 mol) 2-Chlormethylchinolin Hydrochlorid wird weitere 8 h bei 100°C gerührt. Das Lösemittel wird anschließend zum größten Teil im Vakuum abdestilliert. Der Rückstand wird in Ethylacetat aufgenommen, die organische Phase mit Wasser extrahiert, über Natriumsulfat getrocknet und im Vakuum eingeengt. Das Rohprodukt wird säulenchromatographisch gereinigt (Dichlormethan / Methanol, 50:1).
Ausbeute: 14,8 g (63,9% der Theorie)
Fp.: 90°C

### Beispiel 2

### 2-[2-Brom-4-(chinolin-2-yl-methoxy)phenyl]-2-cyclopentyl-essigsäuremethylester

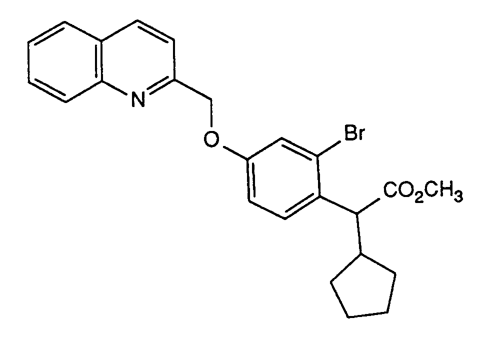

Zu einer Lösung aus 5,9 g (0,015 mol) der Verbindung aus Beispiel 1 und 4,6 g (0,031 mol) Cyclopentylbromid in 20 ml Dimethylformamid wird bei 0°C unter Argonatmosphäre eine Lösung aus 3,77 g (0,034 mol) Kalium-tert.butylat getropft. Anschließend wird 10 h bei Raumtemperatur gerührt. Der Reaktionsansatz wird auf Eiswasser gegossen. Es wird mit Ethylacetat extrahiert, die organische Phase über Natriumsulfat getrocknet und im Vakuum eingeengt. Der Rückstand wird über Kieselgel 60 chromatographiert (Petrolether / Ether, 1:1).
Ausbeute: 5,6 g (80,6% der Theorie), Öl

### Beispiel 3 und Beispiel 4

### 2-[2-Allyl-4-(chinolin-2-yl-methoxy)phenyl]-2-cyclopentylessigsäuremethylester (Beispiel 3)

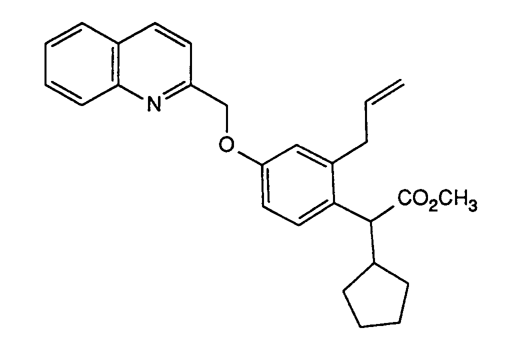

### 2-[4-(Chinolin-2-yl-methoxy)-2-cyclopropylphenyl]-2-cyclopentylessigsäuremethylester (Beispiel 4)

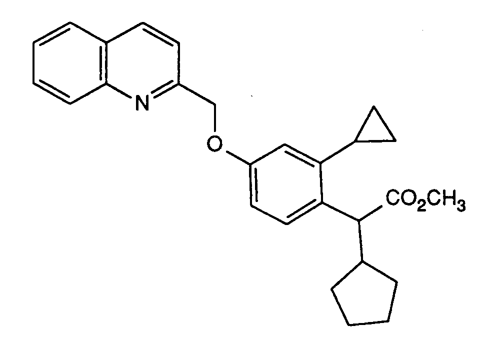

Ein Reaktionsgemisch aus 16 g (0,035 mol) 2-[2-Brom-4-(chinolin-2-yl-methoxy)-phenyl]-2-cyclopentylessigsäuremethylester, 13,2 g (0,039 mol) Allyltributylzinn und 1,6 g (1,4 mmol) Tetrakis(triphenylphosphin)-palladium(0) in 160 ml Toluol p.a. wird 19 h unter Argon und unter Lichtausschluß bei 120°C gerührt. Anschließend wird vom Feststoff abfiltriert und im Vakuum eingeengt. Das Produktgemisch wird säulenchromatographisch gereinigt (Petrolether / Ether 1:1), die Produkte werden auf dieser Stufe nicht getrennt.
Ausbeute: 11,1 g ((3) Allyl- /(4) Cyclopropyl, 7:3) (73,5% der Theorie)

### Beispiel 5

### 2-Allyl-4-(chinolin-2-yl-methoxy)phenylessigsäuremethylester

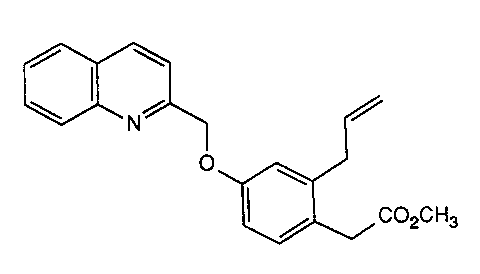

In Analogie zur Vorschrift des Beispiels 3 wird die Titelverbindung aus 15,2 g (0,040 mol) der Verbindung aus Beispiel 1 und 14,4 g (0,044 mol) Allyltributylzinn in Gegenwart von 1,8 g (1,6 mmol) Tetrakis(triphenylphosphin)-palladium(0) hergestellt.
Ausbeute: 8,7 g (62,5% der Theorie), Öl

### Beispiel 6 und Beispiel 4

### 2-[4-(Chinolin-2-yl-methoxy)-2-propylphenyl]-2-cyclopentylessigsäuremethylester (Beispiel 6)

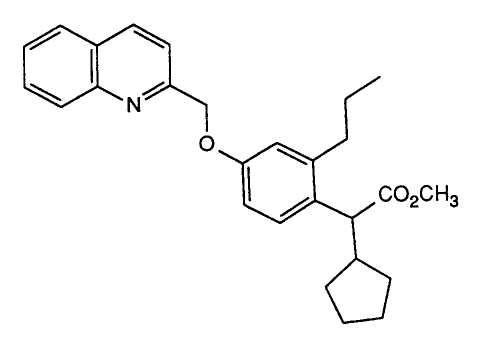

### 2-[4-(Chinolin-2-yl-methoxy)-2-cyclopropylphenyl]-2-cyclopentylessigsäuremethylester (Beispiel 4)

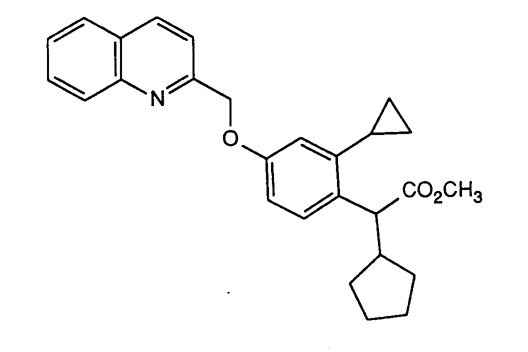

11,1 g des Produktgemisches aus den Beispielen 3 und 4 werden in 190 ml Methanol gelöst und in Gegenwart von 1,5 g Palladium / Kohle (10%) 4 h bei 1,5 bar hydriert. Der Katalysator wird abfiltriert, das Filtrat im Vakuum eingeengt und das Produktgemisch über Kieselgel 60 (Petrolether / Ethylacetat, 10:1) chromatographiert.
Ausbeute: 5,9 g (Beispiel 6)
Ausbeute: 2,5 g (Beispiel 4)

### Beispiel 7

### 4-(Chinolin-2-yl-methoxy)-2-propyl-phenylessigsäuremethylester

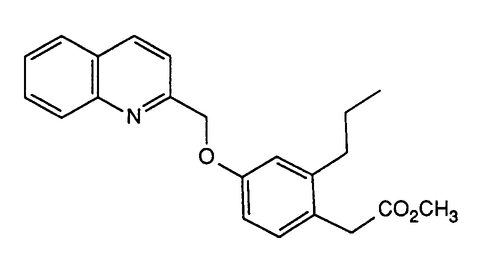

8,7 g (0,025 mol) der Verbindung aus Beispiel 5 werden analog zur Vorschrift der Beispiele 6 und 4 in Gegenwart von 1,3 g Palladium / Kohle (10%) hydriert.
Ausbeute: 3,6 g (42,3% der Theorie), Öl

### Beispiel 8

### 2-[2-Brom-4-(chinolin-2-yl-methoxy)phenyl]-2-cycloheptylessigsäuremethylester

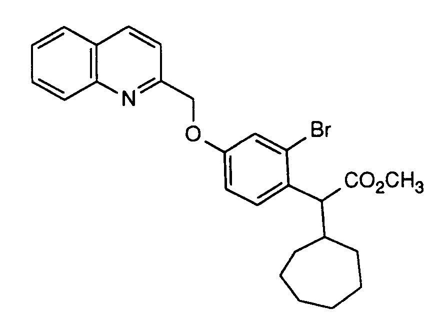

In Analogie zur Vorschrift des Beispiels 2 wird die Titelverbindung aus 23,6 g (0,061 mol) der Verbindung aus Beispiel 1, 21,6 g (0,122 mol) Cycloheptylbromid und 15 g (0,122 mol) Kalium-tert.butylat hergestellt.
Ausbeute: 16,4 g (55,6% d. Theorie)

### Beispiel 9

### 2-[4-(Chinolin-2-yl-methoxy)-2-vinylphenyl]-2-cyclopentylessigsäuremethylester

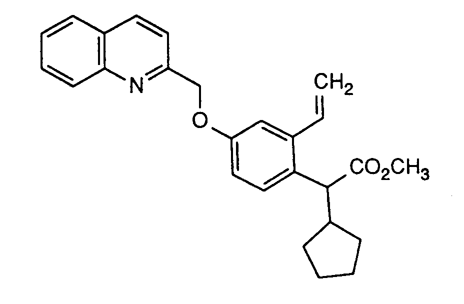

Ein Gemisch aus 2,3 g (5 mmol) der Verbindung aus Beispiel 2, 1,6 g (5 mmol) Tributylvinylzinn und 223 mg (0,2 mmol) Tetrakis(triphenylphosphin)palladium(0) in 40 ml Toluol p.a. wird 15 h unter Argonatmosphäre und unter Lichtausschluß unter Rückfluß erhitzt. Anschließend wird vom Feststoff abfiltriert und im Vakuum eingeengt. Es folgt eine säulenchromtographische Reinigung an Kieselgel 60 mit Petrolether / Ether (1:1). Das erhaltene Produkt ist mit Zinnsalzen verunreinigt und wird direkt weiter umgesetzt.
Ausbeute: 2,5 g Rohprodukt, Öl

### Beispiel 10

### 2-[4-(Chinolin-2-yl-methoxy)-2-ethylphenyl]-2-cyclopentylessigsäuremethylester

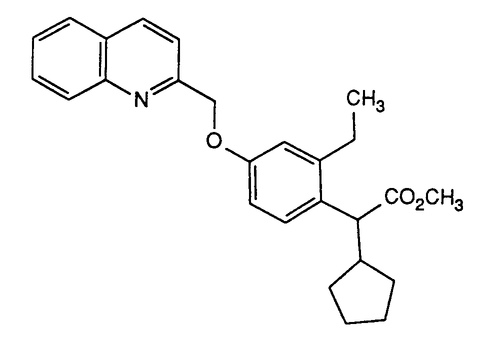

4 g (0,01 mol) der Verbindung aus Beispiel 9 werden in 10 ml Dichlormethan p.a. und 70 ml Methanol p.a. gelöst und 6 h bei 2 bar in Gegenwart von 800 mg Pd-Kohle (10%) hydriert. Es wird vom Katalysator abfiltriert, das Filtrat auf 70 ml Volumen eingeengt und erneut 4 h bei 2 bar in Gegenwart von 800 mg Pd-Kohle (10%) hydriert. Nach Filtration vom Katalysator wird im Vakuum eingeengt und der Rückstand an Kieselgel 60 chromatographiert.
Ausbeute: 1,1 g (27,4% d. Theorie), Öl

### Beispiel 11

### 2-[4-(Chinolin-2-yl-methoxy)-2-vinylphenyl]-2-cycloheptylessigsäuremethylester

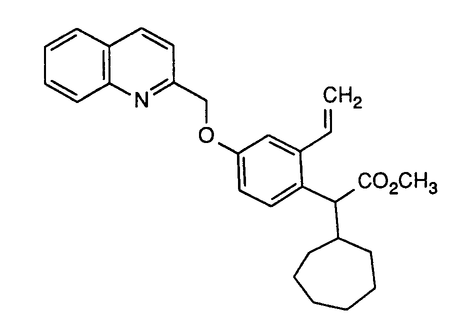

In Analogie zur Vorschrift des Beispiels 9 wird die Titelverbindung aus 7,2 g (0,015 mol) der Verbindung aus Beispiel 8, 4,8 g (0,015 mol) Tributylvinylzinn in Gegenwart von 670 mg (0,006 mol) Tetrakis(triphenylphosphin)-palladium(0) hergestellt
Ausbeute: 6,8 g Rohprodukt (mit Zinnsalzen verunreinigt). Das Rohprodukt wird direkt weiter umgesetzt.

### Beispiel 12

### 2-[4-(Chinolin-2-yl-methoxy)-2-phenylethinylphenyl]-2-cyclopentylessigsäuremethylester

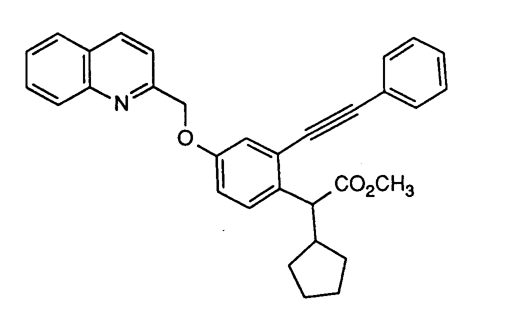

Ein Gemisch aus 9,8 g (0,022 mol) der Verbindung aus Beispiel 2, 20 g (0,054 mol) Phenylethinyltributylzinn und 2,3 g (2 mmol) Tetrakis(triphenylphosphin)-palladium(0) in 80 ml Toluol p.a. wird 36 h unter Argonatmosphäre und unter Lichtausschluß unter Rückfluß erhitzt. Es wird im Vakuum eingeengt und der Rückstand säulenchromatographisch gereinigt (Petrolether / Ether, 1:1).
Ausbeute: 9,0 g (87,7% der Theorie), Öl

### Beispiel 13

### 4-(Chinolin-2-yl-methoxy)-2-isobutylphenylessigsäuremethylester

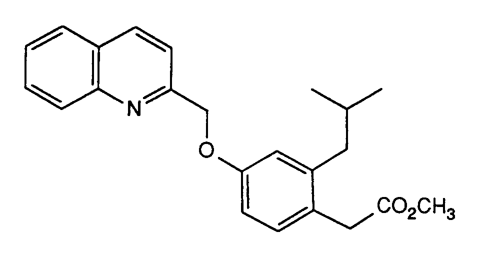

In Analogie zur Vorschrift des Beispiels 1 wird die Titelverbindung aus 1,7 g (7,7 mmol) 4-Hydroxy-2-isobutylphenylessigsäuremethylester, 2,64 g (0,019 mol) Kaliumcarbonat und 1,63 g (7,7 mmol) 2-Chlormethylchinolin Hydrochlorid hergestellt.
Ausbeute: 2,17 g (78% der Theorie), Öl

### Beispiel 14

### 2-[4-(Chinolin-2-yl-methoxy)-2-isobutylphenyl]-2-cycloheptylessigsäuremethylester

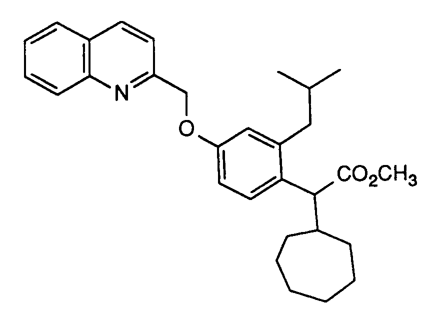

In Analogie zur Vorschrift des Beispiels 1 wird die Titelverbindung aus 1,59 g (5 mmol) 2-(4-Hydroxy-2-isobutylphenyl)-2-cycloheptylessigsäuremethylester, 1,66 g (12 mmol) Kaliumcarbonat und 1,07 g (5 mmol) 2-Chlormethylchinolin Hydrochlorid hergestellt.
Ausbeute: 2 g (89% der Theorie), Öl

### Beispiel 15

### 2-[2-Brom-4-(chinolin-2-yl-methoxy)phenyl]-2-cyclopentylessigsäure

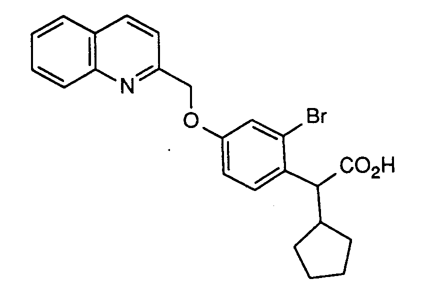

2,2 g (4,8 mmol) der Verbindung aus Beispiel 2 werden mit 50 ml Methanol und 8 ml 1 N NaOH versetzt. Der Reaktionsansatz wird 20 h unter Rückfluß erhitzt, anschließend im Vakuum eingeengt, in Wasser / Diethylether aufgenommen und mit Diethylether gewaschen. Die wäßrige Phase wird mit 2 N Salzsäure angesäuert und mit Diethylether extrahiert. Nach dem Verdampfen des Lösemittels im Vakuum wird das Rohprodukt an Kieselgel 60 chromatographiert (Dichlormethan / Methanol, 9:1).
Ausbeute: 1,85 g (86,8% der Theorie)
Fp.: 73-75°C

### Beispiel 16

### 2-[4-(Chinolin-2-yl-methoxy)-2-isobutylphenyl]-2-cycloheptylessigsäure

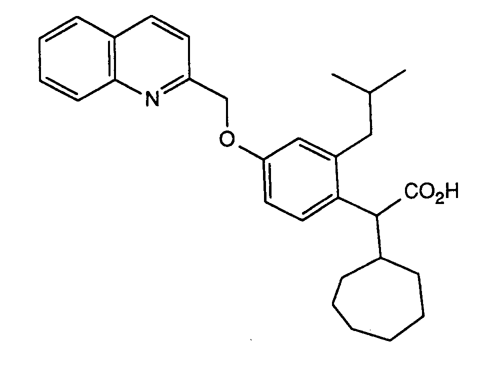

Ein Gemisch aus 2 g (4,3 mmol) der Verbindung aus Beispiel 14, 12 ml Isopropanol und 12 ml 1 N NaOH wird 12 h unter Rückfluß erhitzt. Anschließend wird im Vakuum eingeengt, mit Wasser und Diethylether versetzt und mit Diethylether extrahiert. Die organische Phase wird im Vakuum eingeengt und das zurückgewonnene Edukt erneut zur Verseifung eingesetzt. Die vereinigten wäßrigen Phasen werden mit 2 N Salzsäure angesäuert und mit Ethylacetat extrahiert. Die organische Phase wird über Natriumsulfat getrocknet und im Vakuum eingeengt.

Das Rohprodukt wird in Methanol umkristallisiert. Es werden 3 Reaktionscyclen zur vollständigen Verseifung durchgeführt.
Ausbeute: 1,45 g (76,7% der Theorie)
Fp.: 178-180°C

In Analogie zu den Vorschriften der Beispiele 15 und 16 werden die in Tabelle 1 aufgeführten Verbindungen hergestellt.

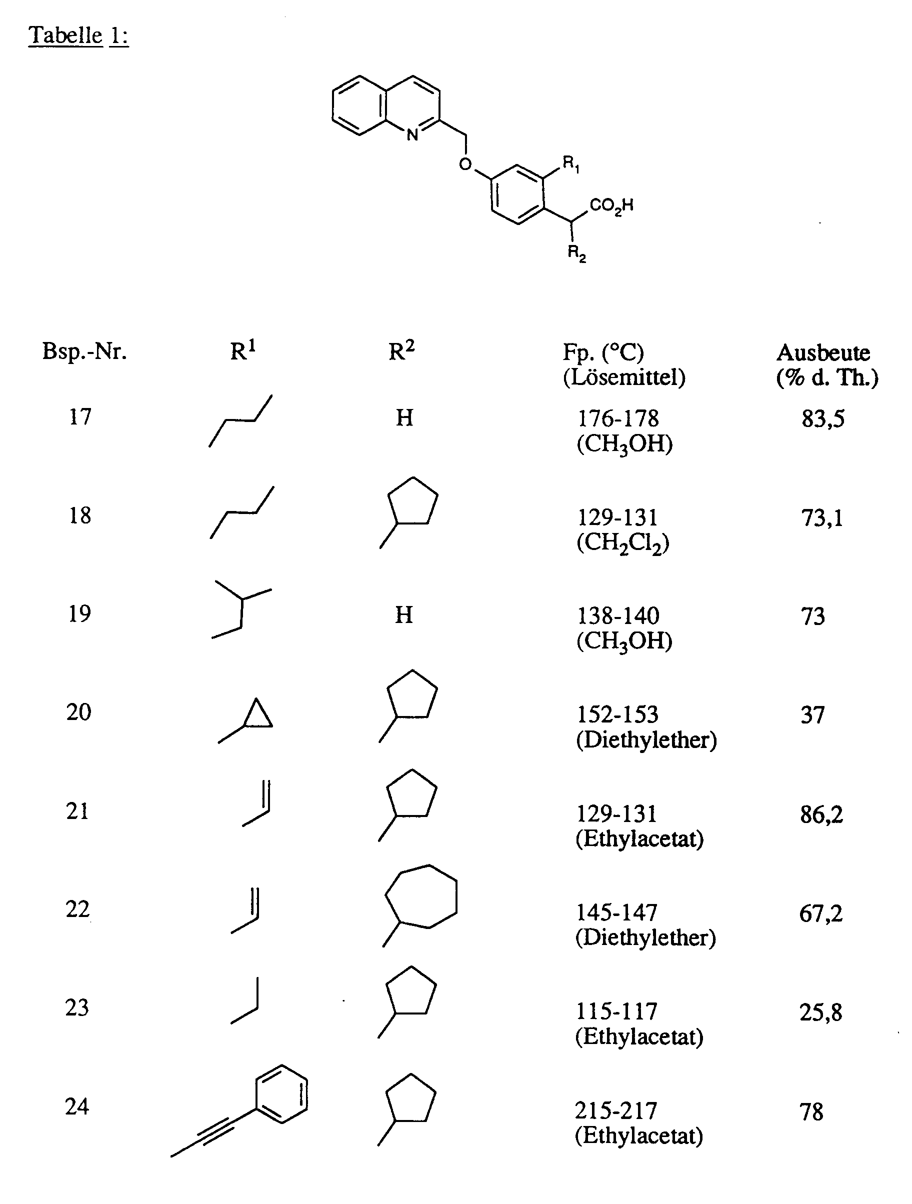

### Beispiel 25

### N-{2-[2-Brom-4-(chinolin-2-yl-methoxy)phenyl]-2-cyclopentylacetyl}-methansulfonamid

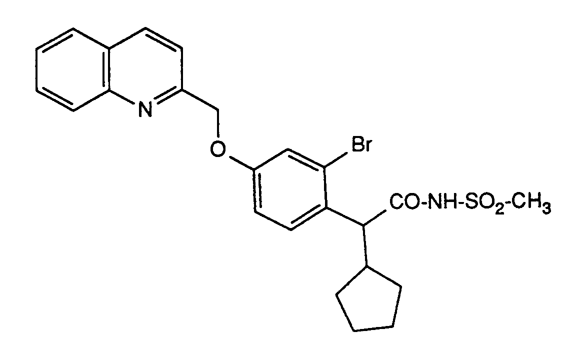

Eine Lösung aus 1,2 g (2,64 mmol) der Verbindung aus Beispiel 15, 280 mg (2,9 mmol) Methansulfonamid, 0,76 g (3,96 mmol) N-(3-Dimethylamino-propyl)-N'-ethylcarbodiimid Hydrochlorid und 0,35 g (2,9 mmol) 4-Dimethylaminopyridin in 60 ml Dichlormethan p.a. wird 20 h bei Raumtemperatur gerührt. Anschließend wird die organische Phase mit Wasser gewaschen, über Natriumsulfat getrocknet und in Vakuum eingeengt. Der Rückstand wird säulenchromatographisch an Kieselgel 60 gereinigt (Dichlormethan / Methanol, 50:1).
Ausbeute: 0,9g (65,9% der Theorie), Öl

### Beispiel 26

### N-{2-[4-(Chinolin-2-yl-methoxy)-2-propylphenyl]acetyl}-N-methyl-trifluormethansulfonamid

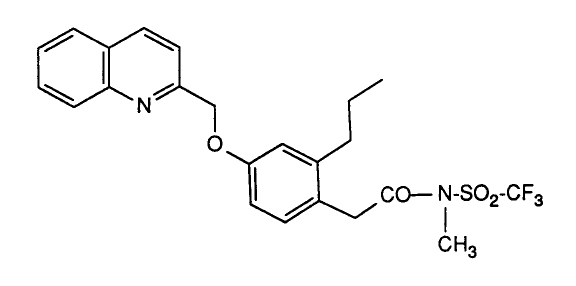

Zu einer Suspension aus 0,67 g (2 mmol) 2-[4-(Chinolin-2-yl-methoxy)-2-propylphenyl]essigsäure und 0,55 ml (4 mmol) Triethylamin in 20 ml Tetrahydrofüran p.a. werden bei 0°C 0,37 ml (4,8 mmol) Methansulfonsäurechlorid getropft. Nach 15 min wird bei 0°C eine Lösung aus 0,59 g (3,6 mmol) N-methyltrifluormethansulfonamid und 0,49 g (4 mmol) 4-Dimethylaminopyridin in 5 ml Tetrahydrofuran p.a. zugetropft. Der Reaktionsansatz wird 20 h bei Raumtemperatur gerührt, anschließend in Eiswasser gegeben und mit Ethylacetat extrahiert. Die organische Phase wird über Natriumsulfat getrocknet und im Vakuum eingeengt. Es folgt eine säulenchromatographische Reinigung der Rückstandes an Kieselgel 60 (Dichlormethan / Methanol, 20:1).
Ausbeute: 0,73 g (76% der Theorie)
Fp.: 111-112°C

In Analogie zu den Vorschriften der Beispiele 25 und 26 werden die in Tabelle 2 aufgeführten Verbindungen hergestellt.

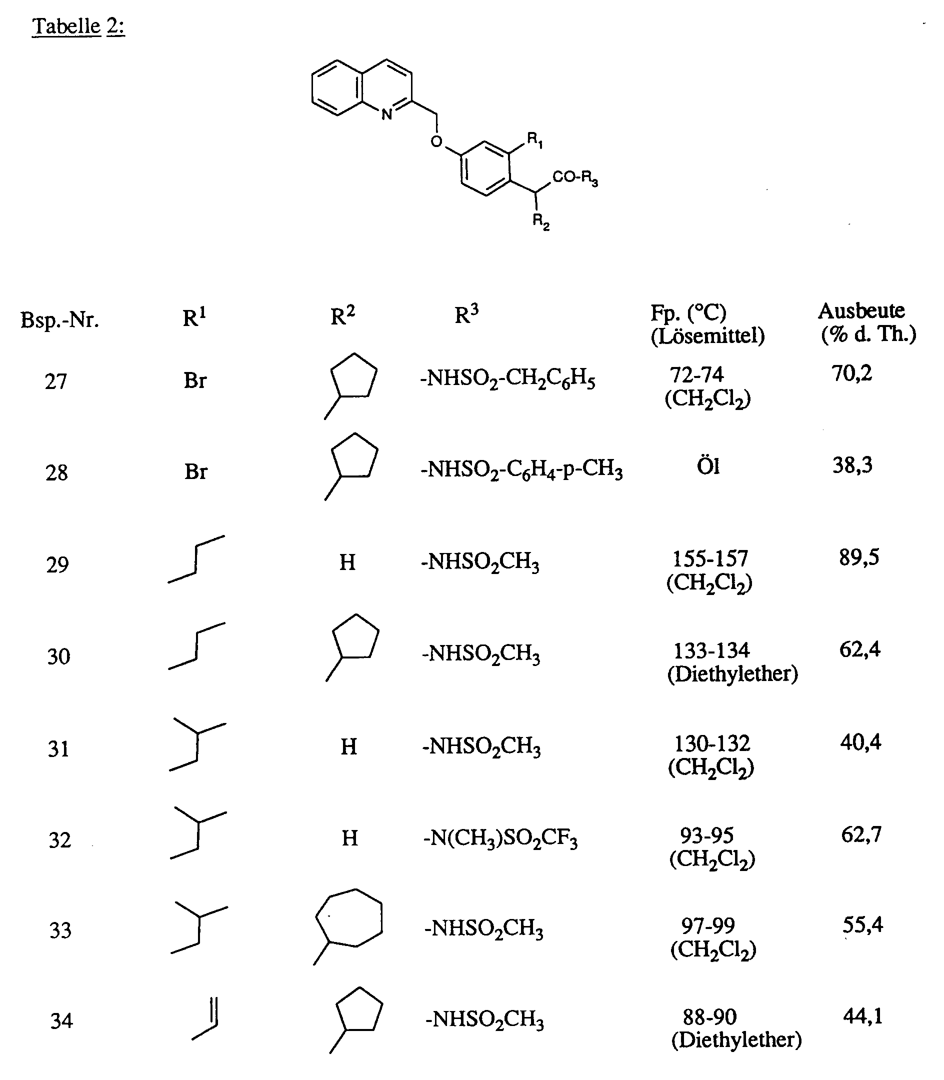

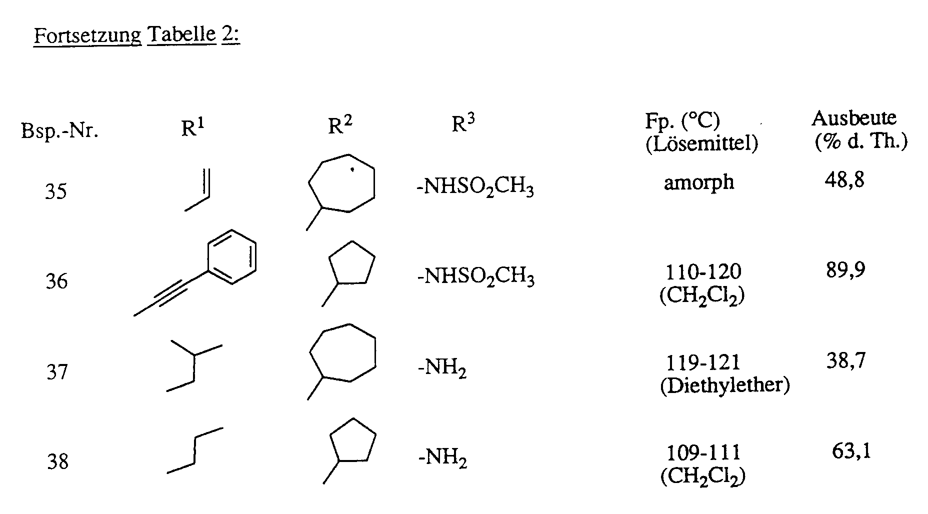

## Patentansprüche

1. 2-Substituierte Chinolylmethoxy-phenylessigsäure - und benzylacyl-derivate der allgemeinen Formel (I) 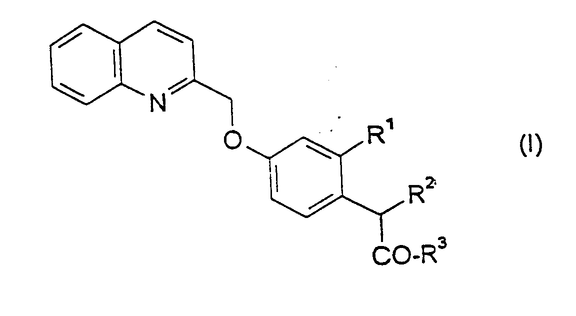 in welcher
R¹ für Halogen, Cyano, Nitro, Azido, Hydroxy, Carboxy, Trifluormethyl, Trifluormethoxy oder Trifluormethylthio steht, oder für geradkettiges oder verzweigtes Alkyl, Alkenyl oder Alkinyl mit jeweils bis zu 8 Kohlenstoffatomen steht, die gegebenenfalls durch Phenyl oder Cycloalkyl mit 3 bis 8 Kohlenstoffatomen substituiert sind, oder
für Cycloalkyl mit 3 bis 8 Kohlenstoffatomen oder Phenyl steht, oder für geradkettiges oder verzweigtes Alkoxy oder Alkoxycarbonyl mit jeweils bis zu 6 Kohlenstoffatomen steht,
R² für Wasserstoff oder für geradkettiges oder verzweigtes Alkyl mit bis zu 6 Kohlenstoffatomen steht, oder
für Cycloalkyl mit 3 bis 12 Kohlenstoffatomen steht,
R³ für Hydroxy, für geradkettiges oder verzweigtes Alkoxy mit bis zu 8 Kohlenstoffatomen oder Phenyl steht, oder
für eine Gruppe der Formel -NR⁴SO₂R⁵ oder -NR⁶R⁷ steht,
worin
R⁴, R⁶ und R⁷ gleich oder verschieden sind und Wasserstoff, geradkettiges oder verzweigtes Alkyl mit bis zu 6 Kohlenstoffatomen, Phenyl oder Benzyl bedeuten,
R⁵ Trifluormethyl oder Phenyl bedeutet, das gegebenenfalls durch Halogen, Cyano, Hydroxy, Nitro, Trifluormethyl, Trifluormethoxy, Trifluormethylthio oder durch geradkettiges oder verzweigtes Alkyl oder Alkoxy mit jeweils bis zu 6 Kohlenstoffatomen substituiert ist, oder
geradkettiges oder verzweigtes Alkyl mit bis zu 8 Kohlenstoffatomen bedeutet, das gegebenenfalls durch Phenyl substituiert ist, das seinerseits durch Halogen, Cyano, Nitro, Trifluormethyl, Trifluormethoxy, Trifluormethylthio, Hydroxy oder durch geradkettiges oder verzweigtes Alkyl oder Alkoxy mit jeweils bis zu 6 Kohlenstoffatomen substituiert sein kann
und deren Salze.

2. 2-Substituierte Chinolylmethoxy-phenylessigsäure-und -benzylacyl-derivate der Formel nach Anspruch 1,
wobei
R¹ für Fluor, Chlor, Brom, Iod, Cyano, Nitro, Azido, Hydroxy, Carboxy, Trifluormethyl oder Trifluormethoxy steht, oder
für geradkettiges oder verzweigtes Alkyl, Alkenyl oder Alkinyl mit jeweils bis zu 6 Kohlenstoffatomen sieht, die gegebenenfalls durch Phenyl, Cyclopropyl, Cyclopentyl oder Cyclohexyl substituiert sind, oder
für Cyclopropyl, Cyclobutyl, Cyclopentyl, Cyclohexyl oder Cycloheptyl oder Phenyl sieht, oder
für geradkettiges oder verzweigtes Alkoxy oder Alkoxycarbonyl mit jeweils bis zu 4 Kohlenstoffatomen steht,
R² für Wasserstoff oder geradkettiges oder verzweigtes Alkyl mit bis zu 4 Kohlenstoffatomen steht, oder
für Cyclopropyl, Cyclobutyl, Cyclopentyl, Cyclohexyl, Cycloheptyl oder Cyclooctyl steht,
R³ für Hydroxy oder für geradkettiges oder verzweigtes Alkoxy mit bis zu 6 Kohlenstoffatomen oder Phenyl steht, oder
für eine Gruppe der Formel -NR⁴SO₂R⁵ oder -NR⁶R⁷ steht,
worin
R⁴, R⁶ und R⁷ gleich oder verschieden sind und Wasserstoff oder geradkettiges oder verzweigtes Alkyl mit bis zu 4 Kohlenstoffatomen bedeuten,
R⁵ Trifluormethyl oder Phenyl bedeutet, das gegebenenfalls durch Fluor, Chlor, Brom, Iod, Cyano oder durch geradkettiges oder verzweigtes Alkyl oder Alkoxy mit jeweils bis zu 4 Kohlenstoffatomen substituiert ist, oder
geradkettiges oder verzweigtes Alkyl mit bis zu 6 Kohlenstoffatomen bedeutet, das gegebenenfalls durch Phenyl substituiert ist, das seinerseits durch Fluor, Chlor, Brom, Trifluormethyl oder durch geradkettiges oder verzweigtes Alkyl oder Alkoxy mit jeweils bis zu 4 Kohlenstoffatomen substituiert sein kann
und deren Salze.

3. 2-Substituierte Chinolylmethoxy-phenylessigsäure und -benzylacyl-derivate der Formel nach Anspruch 1,
wobei
R¹ für Fluor, Chlor, Brom, Nitro, Azido oder Trifluormethoxy steht, oder für geradkettiges oder verzweigtes Alkyl, Alkenyl oder Alkinyl mit jeweils bis zu 4 Kohlenstoffatomen steht, die gegebenenfalls durch Phenyl oder Cyclopropyl substituiert sind, oder
für Cyclopropyl, Cyclopentyl oder Cyclohexyl steht,
R² für Wasserstoff oder für geradkettiges oder verzweigtes Alkyl mit bis Zu 3 Kohlenstoffatomen steht, oder
für Cyclopentyl, Cyclohexyl oder Cycloheptyl steht,
R³ für Hydroxy oder für geradkettiges oder verzweigtes Alkoxy mit bis zu 4 Kohlenstoffatomen steht, oder
für eine Gruppe der Formel -NR⁴SO₂R⁵ oder -NR⁶R⁷ steht,
worin
R⁴, R⁶ und R⁷ gleich oder verschieden sind und Wasserstoff oder Methyl bedeuten,
R⁵ Trifluormethyl oder Phenyl bedeutet, das gegebenenfalls durch Fluor, Chlor, Brom, Jod, Methoxy, Methyl oder Trifluormethyl substituiert ist, oder
geradkettiges oder verzweigtes Alkyl mit bis zu 4 Kohlenstoffatomen bedeutet, das gegebenenfalls durch Phenyl substituiert ist, das seinerseits durch Fluor, Chlor, Brom, Methyl oder Methoxy substituiert sein kann
und deren Salze.

4. 2-Substituierte Chinolylmethoxy- phenylessigsäure- und -benzylacyl-derivate nach Anspruch 1 bis 3 zur therapeutischen Anwendung.

5. Verfahren zur Herstellung von 2-substituierten Chinolylmethoxy-phenylessigsäure - und -benzylacyl-derivate nach Anspruch 1 bis 3, dadurch gekennzeichnet, daß man
[A] Phenole der allgemeinen Formel (II) 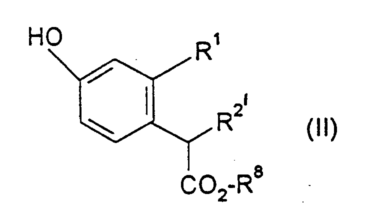 in welcher
R^{2'} die oben angegebene Bedeutung von R² hat aber nicht für Wasserstoff sieht,
R⁸ für C₁-C₄-Alkyl steht,
mit 2-Halogenmethylchinolinen der allgemeinen Formel (III) 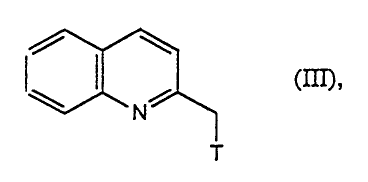 in welcher
T für Halogen, vorzugsweise für Chlor oder Brom steht,
in inerten Lösemitteln verethert,
oder
[B] Phenole der allgemeinen Formel (IIa) 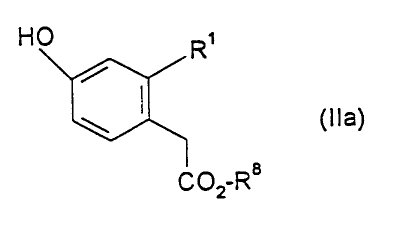 zunächst durch Umsetzung mit den Verbindungen der allgemeinen Formel (III) in inerten Lösemitteln in die Verbindungen der allgemeinen Formel (Ia) 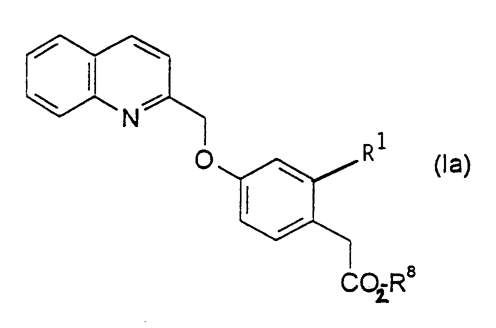 überführt,
und diese anschließend mit Verbindungen der allgemeinen Formel (IV)
R^{2'}-W (IV),
in welcher
W für Chlor, Brom oder Jod steht,
in inerten Lösemitteln alkyliert,
und im Fall der Säuren (R³ = OH) die Ester verseift,
im Fall, daß R³ für die Gruppe der Formel -NR⁴SO₂R⁵ oder -NR⁶R⁷ steht, die Säuren (R³ = OH), gegebenenfalls unter vorgeschalteter Aktivierung, diese entweder mit den entsprechenden Sulfonamiden der Formel (V), den Aminen oder Ammoniak der Formel (VI)
NHR⁴SO₂-R⁵ (V)
oder
HNR⁶R⁷ (VI),
sulfoamidiert bzw. amidiert,
im Fall, daß R¹ für Alkenyl oder Alkinyl sieht, ausgehend von den entsprechenden Halogenverbindungen der allgemeinen Formel (Ia / R¹ = Halogen, vorzugsweise Brom) mit Verbindungen der allgemeinen Formel (VII)
(C₄H₉)₃Sn-R^{1'} (VII),
in welcher
R^{1'} für Alkenyl oder Alkinyl mit bis zu 8 C-Atomen steht,
in Anwesenheit von Palladium(0)-Katalysatoren, vorzugsweise Tetrakis-(triphenylphosphin)palladium(0), umsetzt,
und im Fall, daß R¹ = (C₁-C₈)-Alkyl, gegebenenfalls anschließend nach üblichen Methoden hydriert,
und im Fall der Enantiomere die entsprechenden enantiomerenreinen Säuren (I / R³ = OH) nach üblicher Methode trennt,
und den Substituenten R¹ gegebenenfalls auf jeder der oben aufgeführten Stufen durch weitere übliche Methoden einführt oder variiert.

6. Arzneimittel enthaltend mindestens ein 2-substituiertes Chinolylmethyoxyphenylessigsäure- und benzylacyl-derivate nach Anspruch 1 bis 3.

7. Arzneimittel nach Anspruch 8 enthaltend die 2-substituierten Chinolylmethoxy-phenylessigsäure- und -benzylacyl-derivate in einer Menge von 0,1 bis 99,5 Gew.-% der Gesamtmischung.

8. Verwendung der 2-substituierten Chinolylmethoxy-phenylessigsäure - und -benzylacyl-derivate nach Anspruch 1 bis 3 zur Herstellung von Arzneimitteln.

## Claims

1. Substituted quinol-2-yl-methoxy-phenylacetic acid and benzylacyl derivatives of the general formula (I) 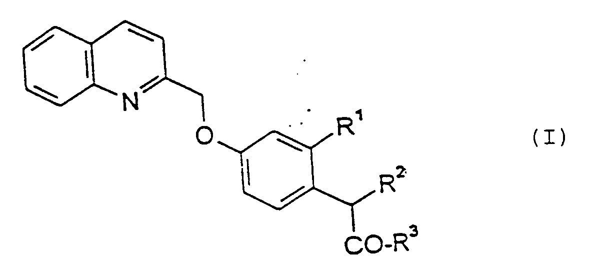 in which
R¹ represents halogen, cyano, nitro, azido, hydroxyl, carboxyl, trifluoromethyl, trifluoromethoxy or trifluoromethylthio, or
represents straight-chain or branched alkyl, alkenyl or alkinyl with in each case up to 8 carbon atoms, which are optionally substituted by phenyl or cycloalkyl with 3 to 8 carbon atoms, or
represents cycloalkyl with 3 to 8 carbon atoms or phenyl, or
represents straight-chain or branched alkoxy or alkoxycarbonyl with in each case up to 6 carbon atoms,
R² represents hydrogen or represents straight-chain or branched alkyl with up to 6 carbon atoms, or
represents cycloalkyl with 3 to 12 carbon atoms,
R³ represents hydroxyl, or represents straight-chain or branched alkoxy with up to 8 carbon atoms or phenyl, or
represents a group of the formula -NR⁴SO₂R⁵ or -NR⁶R⁷,
in which
R⁴, R⁶ and R⁷ are identical or different and represent hydrogen, straight-chain or branched alkyl with up to 6 carbon atoms, phenyl or benzyl,
R⁵ represents trifluoromethyl or phenyl, which is optionally substituted by halogen, cyano, hydroxyl, nitro, trifluoromethyl, trifluoromethoxy or trifluoromethylthio, or by straight-chain or branched alkyl or alkoxy with in each case up to 6 carbon atoms, or represents straight-chain or branched alkyl with up to 8 carbon atoms, which is optionally substituted by phenyl, which in turn may be substituted by halogen, cyano, nitro, trifluoromethyl, trifluoromethoxy, trifluoromethylthio or hydroxyl or by straight-chain or branched alkyl or alkoxy with in each case up to 6 carbon atoms,
and their salts.

2. Substituted quinol-2-yl-methoxy-phenylacetic acid and benzylacyl derivatives of the formula according to Claim 1, where
R¹ represents fluorine, chlorine, bromine, iodine, cyano, nitro, azido, hydroxyl, carboxyl, trifluoromethyl or trifluoromethoxy, or
represents straight-chain or branched alkyl, alkenyl or alkinyl with in each case up to 6 carbon atoms, which are optionally substituted by phenyl, cyclopropyl, cyclopentyl or cyclohexyl, or
represents cyclopropyl, cyclobutyl, cyclopentyl, cyclohexyl or cycloheptyl or phenyl, or represents straight-chain or branched alkoxy or alkoxycarbonyl with in each case up to 4 carbon atoms,
R² represents hydrogen or represents straight-chain or branched alkyl with up to 4 carbon atoms, or
represents cyclopropyl, cyclobutyl, cyclopentyl, cyclohexyl, cycloheptyl or cyclooctyl,
R³ represents hydroxyl, or represents straight-chain or branched alkoxy with up to 6 carbon atoms or phenyl, or
represents a group of the formula -NR⁴SO₂R⁵ or -NR⁶R⁷,
in which
R⁴, R⁶ and R⁷ are identical or different and represent hydrogen or straight-chain or branched alkyl with up to 4 carbon atoms,
R⁵ represents trifluoromethyl or phenyl, which is optionally substituted by fluorine, chlorine, bromine, iodine or cyano, or by straight-chain or branched alkyl or alkoxy with in each case up to 4 carbon atoms, or
represents straight-chain or branched alkyl with up to 6 carbon atoms, which is optionally substituted by phenyl, which in turn may be substituted by fluorine, chlorine, bromine or trifluoromethyl, or by straight-chain or branched alkyl or alkoxy with in each case up to 4 carbon atoms,
and their salts.

3. Substituted quinol-2-yl-methoxy-phenylacetic acid and benzylacyl derivatives of the formula according to Claim 1, where
R¹ represents fluorine, chlorine, bromine, nitro, azido or trifluoromethoxy, or
represents straight-chain or branched alkyl, alkenyl or alkinyl with in each case up to 4 carbon atoms, which are optionally substituted by phenyl or cyclopropyl, or
represents cyclopropyl, cyclopentyl or cyclohexyl,
R² represents hydrogen, or represents straight-chain or branched alkyl with up to 3 carbon atoms, or
represents cyclopentyl, cyclohexyl or cycloheptyl,
R³ represents hydroxyl, or represents straight-chain or branched alkoxy with up to 4 carbon atoms, or represents a group of the formula -NR⁴SO₂R⁵ or -NR⁶R⁷,
in which
R⁴, R⁶ and R⁷ are identical or different and represent hydrogen or methyl,
R⁵ represents trifluoromethyl or phenyl, which is optionally substituted by fluorine, chlorine, bromine, iodine, methoxy, methyl or trifluoromethyl, or represents straight-chain or branched alkyl with up to 4 carbon atoms, which is optionally substituted by phenyl, which in turn may be substituted by fluorine, chlorine, bromine, methyl or methoxy,
and their salts.

4. Substituted quinol-2-yl-methoxy-phenylacetic acid and benzylacyl derivatives according to Claims 1 to 3 for therapeutic use.

5. Process for preparing substituted quinol-2-yl-methoxy-phenylacetic acid and benzylacyl derivatives according to Claims 1 to 3, characterized in that
[A] phenols of the general formula (II) 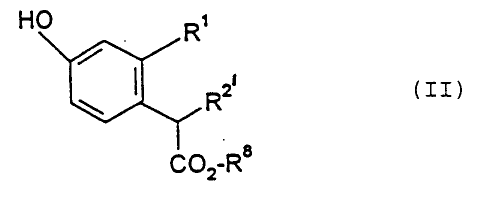 in which
R^{2'} has the abovementioned meaning of R² but does not represent hydrogen, and
R⁸ represents C₁-C₄-alkyl,
are etherified in inert solvents with 2-halogenomethylquinolines of the general formula (III) 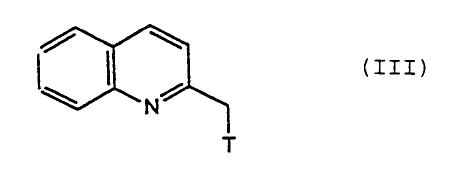 in which
T represents halogen, preferably chlorine or bromine,
or
[B] phenols of the general formula (IIa) 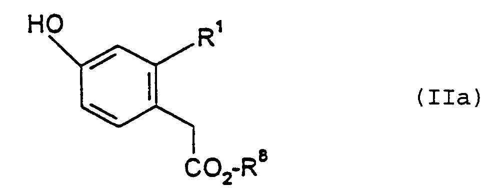 are first converted, by reaction with the compounds of the general formula (III) in inert solvents, into the compounds of the general formula (Ia) 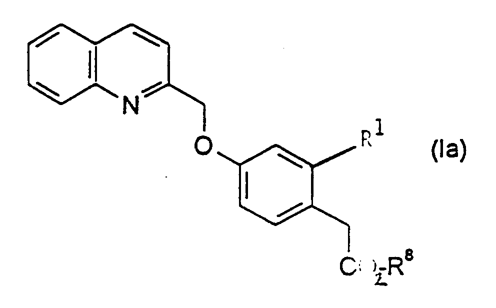 and the latter are subsequently alkylated in inert solvents with compounds of the general formula (IV)
R^{2'}-W (IV)
in which
W represents chlorine, bromine or iodine,
and in the case of the acids (R³ = OH) the esters are hydrolyzed,
and, in the case that R³ represents the group of the formula -NR⁴SO₂R⁵ or -NR⁶R⁷, the acids (R³ = OH), optionally with prior activation, are sulphoamidated or amidated, respectively, with the corresponding sulphonamides of the formula (V) or the amines or ammonia of the formula (VI)
NHR⁴SO₂-R⁵ (V)
or
HNR⁶R⁷ (VI)
and, in the case that R¹ represents alkenyl or alkinyl, reaction is carried out, starting from the corresponding halogeno compounds of the general formula (Ia / R¹ = halogen, preferably bromine), with compounds of the general formula (VII)
(C₄H₉)₃Sn-R^{1'} (VII)
in which
R^{1'} represents alkenyl or alkinyl having up to 8 C atoms,
in the presence of palladium(0) catalysts, preferably tetrakis(triphenylphosphine)palladium(0),
and, in the case that R¹ = (C₁-C₈)-alkyl, hydrogenation is optionally carried out subsequently according to customary methods,
and, in the case of the enantiomers, the corresponding enantiomerically pure acids (I/R³ = OH) are separated by a customary method,
and the substituent R¹ is optionally introduced or modified by further customary methods at each of the above-listed stages.

6. Medicament containing at least one substituted quinol-2-yl-methoxy-phenylacetic acid and benzylacyl derivative according to Claims 1 to 3.

7. Medicament according to Claim 8 containing the substituted quinol-2-yl-methoxy-phenylacetic acid and benzylacyl derivatives in a quantity from 0.1 to 99.5% by weight of the complete mixture.

8. Use of the substituted quinol-2-yl-methoxy-phenylacetic acid and benzylacyl derivatives according to Claims 1 to 3 for preparing medicaments.

## Revendications

1. Dérivés d'acide quinolylméthoxy-phénylacétique et benzylacylés substitués en position 2 répondant à la formule générale (I) 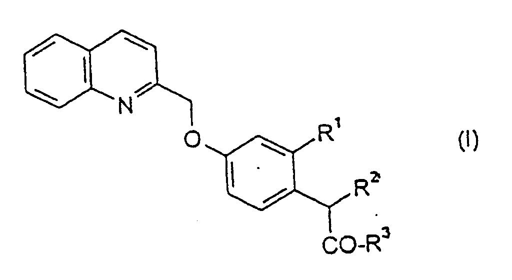 dans laquelle
R¹ représente un atome d'halogène, un groupe cyano, un groupe nitro, un groupe azido, un groupe hydroxyle, un groupe carboxyle, un groupe trifluorométhyle, un groupe trifluorométhoxy ou un groupe trifluorométhylthio, ou représente un groupe alkyle, un groupe alcényle ou un groupe alcynyle à chaîne droite ou ramifiée, chacun de ces groupes contenant jusqu'à 8 atomes de carbone, qui portent éventuellement un ou plusieurs substituants identiques ou différents phényle ou cycloalkyle contenant de 3 à 8 atomes de carbone, ou
représente un groupe cycloalkyle contenant de 3 à 8 atomes de carbone ou un groupe phényle, ou encore
représente un groupe alcoxy ou un groupe alcoxycarbonyle à chaîne droite ou ramifiée, chacun de ces groupes contenant jusqu'à 6 atomes de carbone,
R² représente un atome d'hydrogène ou représente un groupe alkyle à chaîne droite ou ramifiée contenant jusqu'à 6 atomes de carbone, ou encore
représente un groupe cycloalkyle contenant de 3 à 12 atomes de carbone,
R³ représente un groupe hydroxyle; un groupe alcoxy à chaîne droite ou ramifiée contenant jusqu'à 8 atomes de carbone ou encore un groupe phényle, ou
représente un groupe répondant à la formule -NR⁴SO₂R⁵ ou -NR⁶R⁷
dans lesquelles
R⁴, R⁶ et R⁷ sont identiques ou différents et représentent un atome d'hydrogène, un groupe alkyle à chaîne droite ou ramifiée contenant jusqu'à 6 atomes de carbone, un groupe phényle ou un groupe benzyle,
R⁵ représente un groupe trifluorométhyle ou un groupe phényle, qui porte éventuellement un ou plusieurs substituants identiques ou différents halogéno, cyano, hydroxyle, nitro, trifluorométhyle, trifluorométhoxy, trifluorométhylthio ou encore un ou plusieurs substituants identiques ou différents alkyle ou alcoxy à chaînes droites ou ramifiées, chacun de ces substituants contenant jusqu'à 6 atomes de carbone, ou
représente un groupe alkyle à chaîne droite ou ramifiée contenant jusqu'à 8 atomes de carbone, qui porte éventuellement un ou plusieurs substituants identiques ou différents phényle, celui-ci pouvant porter quant à lui un ou plusieurs substituants identiques ou différents halogéno, cyano, nitro, trifluorométhyle, trifluorométhoxy, trifluorométhylthio, hydroxyle ou encore un ou plusieurs substituants identiques ou différents alkyle ou alcoxy à chaînes droites ou ramifiées, chacun de ces substituants contenant jusqu'à 6 atomes de carbone,
et leurs sels.

2. Dérivés d'acide quinolylméthoxy-phénylacétique et benzylacylés substitués en position 2 de la formule selon la revendication 1,
où
R¹ représente un atome de fluor, un atome de chlore, un atome de brome, un atome d'iode, un groupe cyano, un groupe nitro, un groupe azido, un groupe hydroxyle, un groupe carboxyle, un groupe trifluorométhyle ou un groupe trifluorométhoxy, ou
représente un groupe alkyle, un groupe alcényle ou un groupe alcynyle à chaîne droite ou ramifiée, chacun de ces groupes contenant jusqu'à 6 atomes de carbone, qui portent éventuellement un ou plusieurs substituants identiques ou différents phényle, cyclopropyle, cyclopentyle ou cyclohexyle, ou représente un groupe cyclopropyle, un groupe cyclobutyle, un groupe cyclopentyle, un groupe cyclohexyle ou un groupe cycloheptyle ou encore un groupe phényle, ou
représente un groupe alcoxy ou un groupe alcoxycarbonyle à chaîne droite ou ramifiée, chacun de ces groupes contenant jusqu'à 4 atomes de carbone,
R² représente un atome d'hydrogène ou représente un groupe alkyle à chaîne droite ou ramifiée contenant jusqu'à 4 atomes de carbone, ou représente un groupe cyclopropyle, un groupe cyclobutyle, un groupe cyclopentyle, un groupe cyclohexyle, un groupe cycloheptyle ou un groupe cyclooctyle,
R³ représente un groupe hydroxyle ou représente un groupe alcoxy à chaîne droite ou ramifiée contenant jusqu'à 6 atomes de carbone ou encore un groupe phényle, ou encore
représente un groupe répondant à la formule -NR⁴SO₂R⁵ ou -NR⁶R⁷
dans lesquelles
R⁴, R⁶ et R⁷ sont identiques ou différents et représentent un atome d'hydrogène ou un groupe alkyle à chaîne droite ou ramifiée contenant jusqu'à 4 atomes de carbone,
R⁵ représente un groupe trifluorométhyle ou un groupe phényle, qui porte éventuellement un ou plusieurs substituants identiques ou différents fluoro, chloro, bromo, iodo, cyano ou encore un ou plusieurs substituants identiques ou différents alkyle ou alcoxy à chaînes droites ou ramifiées, chacun de ces substituants contenant jusqu'à 4 atomes de carbone, ou représente un groupe alkyle à chaîne droite ou ramifiée contenant jusqu'à 6 atomes de carbone, qui porte éventuellement un ou plusieurs substituants identiques ou différents phényle, celui-ci pouvant porter quant à lui un ou plusieurs substituants identiques ou différents fluoro, chloro, bromo, trifluorométhyle ou encore un ou plusieurs substituants identiques ou différents alkyle ou alcoxy à chaînes droites ou ramifiées, chacun de ces substituants contenant jusqu'à 4 atomes de carbone,
et leurs sels.

3. Dérivés d'acide quinolylméthoxy-phénylacétique et benzylacylés substitués en position 2 de la formule selon la revendication 1,
où
R¹ représente un atome de fluor, un atome de chlore, un atome de brome, un groupe nitro, un groupe azido ou un groupe trifluorométhoxy, ou représente un groupe alkyle, un groupe alcényle ou un groupe alcynyle à chaîne droite ou ramifiée, chacun de ces groupes contenant jusqu'à 4 atomes de carbone, qui portent éventuellement un ou plusieurs substituants identiques ou différents phényle ou cyclopropyle, ou
représente un groupe cyclopropyle, un groupe cyclopentyle ou un groupe cyclohexyle,
R² représente un atome d'hydrogène ou représente un groupe alkyle à chaîne droite ou ramifiée contenant jusqu'à 3 atomes de carbone, ou encore
représente un groupe cyclopentyle, un groupe cyclohexyle ou un groupe cycloheptyle,
R³ représente un groupe hydroxyle ou représente un groupe alcoxy à chaîne droite ou ramifiée contenant jusqu'à 4 atomes de carbone ou encore
représente un groupe répondant à la formule -NR⁴SO₂R⁵ ou -NR⁶R⁷
dans lesquelles
R⁴, R⁶ et R⁷ sont identiques ou différents et représentent un atome d'hydrogène ou un groupe méthyle,
R⁵ représente un groupe trifluorométhyle ou un groupe phényle, qui porte éventuellement un ou plusieurs substituants identiques ou différents fluoro, chloro, bromo, iodo, méthoxy, méthyle ou trifluorométhyle, ou
représente un groupe alkyle à chaîne droite ou ramifiée contenant jusqu'à 4 atomes de carbone, qui porte éventuellement un ou plusieurs substituants identiques ou différents phényle, celui-ci pouvant porter quant à lui un ou plusieurs substituants identiques ou différents fluoro, chloro, bromo, méthyle ou méthoxy,
et leurs sels.

4. Dérivés d'acide quinolylméthoxy-phénylacétique et benzylacylés substitués en position 2 selon les revendications 1 à 3 pour l'application thérapeutique.

5. Procédé pour la préparation de dérivés d'acide quinolylméthoxy-phénylacétique et benzylacylés substitués en position 2 selon les revendications 1 à 3, caractérisé en ce que
[A] on éthérifie dans des solvants inertes des phénols répondant à la formule générale (II) 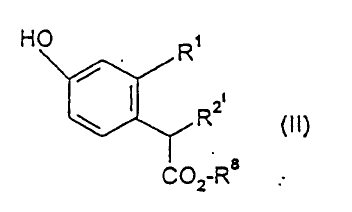 dans laquelle
R^{2'} a la signification indiquée ci-dessus pour R², mais ne représente pas un atome d'hydrogène,
R⁸ représente un groupe alkyle en C₁-C₄,
avec des 2-halogénométhylquinoléines répondant à la formule générale (III) 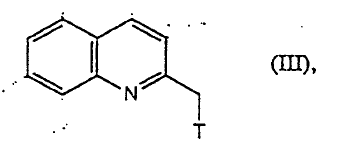 dans laquelle
T représente un atome d'halogène, de préférence un atome de chlore ou un atome de brome,
ou
[B] on transforme d'abord des phénols répondant à la formule générale (IIa) 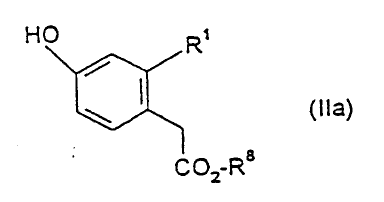 par mise en réaction avec les composés répondant à la formule générale (III) dans des solvants inertes en composés répondant à la formule générale (Ia) 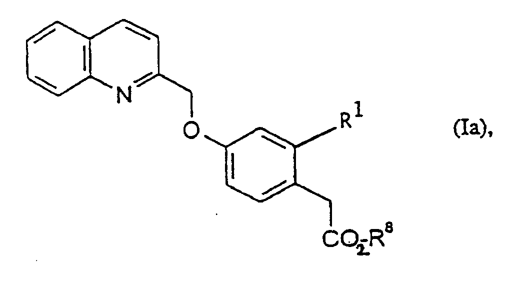 puis, on soumet ces derniers à une alkylation dans des solvants inertes avec des composés répondant à la formule générale (IV)
R^{2'}-W (IV)
dans laquelle
W représente un atome de chlore, un atome de brome ou un atome d'iode,
et, dans le cas des acides (R³=OH), on saponifie les esters,
dans le cas où R³ représente le groupe de formules -NR⁴SO₂R⁵ ou -NR⁶R⁷, on soumet les acides (R³=OH), éventuellement après activation préalable, à une sulfoamidation, respectivement à une amidation, soit avec les sulfonamides correspondants de formule (V), soit avec les amines ou l'ammoniac de formule (VI)
NHR⁴SO₂-R⁵ (V)
ou
NHR⁶R⁷ (VI)
dans le cas où R¹ représente un groupe alcényle ou un groupe alcynyle à partir des composés halogénés correspondants répondant à la formule générale (Ia/R¹ = un atome d'halogène, de préférence un atome de brome), on les fait réagir avec des composés répondant à la formule générale (VII)
(C₄H₉)₃Sn-R^{1'} (VII)
dans laquelle
R^{1'} représente un groupe alcényle ou un groupe alcynyle contenant jusqu'à 8 atomes de carbone,
en présence de catalyseurs de palladium(0), de préférence en présence du tétrakis-(triphénylphosphine)-palladium(0),
et, dans le cas où R¹ représente un groupe alkyle en C₁-C₈, on les soumet ensuite, le cas échéant, à une hydrogénation conformément à des procédés habituels,
et, dans le cas des énantiomères, on sépare les acides correspondants sous forme d'énantiomères purs (I/R³=OH) conformément au procédé habituel,
et on introduit ou on fait varier les substituants R¹ éventuellement à chacune des étapes indiquées ci-dessus, via d'autres procédés habituels.

6. Médicament contenant au moins un dérivé d'acide quinolylméthoxy-phénylacétique et benzylacylé substitué en position 2 selon les revendications 1 à 3.

7. Médicament selon la revendication 8, contenant le dérivé d'acide quinolylméthoxy-phénylacétique et benzylacylé substitué en position 2 en une quantité de 0,1 à 99,5% en poids du mélange total.

8. Utilisation des dérivés d'acide quinolylméthoxy-phénylacétique et benzylacylés substitués en position 2 selon les revendications 1 à 3 pour la préparation de médicaments.
